# EUROPÄISCHE PATENTSCHRIFT

(11) **EP 0 935 691 B1**
(45) Veröffentlichungstag und Bekanntmachung des Hinweises auf die Patenterteilung: **26.05.2004**
(21) Anmeldenummer: 97913175.2
(22) Anmeldetag: 24.10.1997
(51) Int. Cl.: D06L 3/12, C11D 3/42

(54) **OPTISCHER AUFHELLER**
OPTICAL BRIGHTENING AGENT
AGENT DE BLANCHIMENT OPTIQUE

(30) Priorität: 31.10.1996 DE 19645063
(43) Veröffentlichungstag der Anmeldung: 18.08.1999
(73) Patentinhaber: Covion Organic Semiconductors GmbH, 65926 Frankfurt (DE)
(72) Erfinder: SALBECK, Josef, D-65779 Kelkheim (DE); KREUDER, Willi, D-55126 Mainz (DE)
(74) Vertreter: Luderschmidt, Schüler & Partner GbR
(86) Internationale Anmeldenummer: PCT/EP1997/005888
(87) Internationale Veröffentlichungsnummer: WO 1998/018996

(56) Entgegenhaltungen:
- EP-A- 0 676 461
- DE-A- 4 442 050

## Beschreibung

Als optische Aufheller bezeichnet man nach Römpps Chemie Lexikon (Römpp Hermann [Begr.]; Falbe, Jürgen [Hrsg.]; Chemie Lexikon, Georg Thieme Verlag, Stuttgart 1991) solche chemischen Verbindungen, die Vergrauungen und Vergilbungen von Textilien, Papier, Kunststoffen usw. dadurch beseitigen, daß sie, wie Farbstoffe aus der Flotte auf der Faser aufgezogen bzw. in das betreffende Material eingearbeitet, eine Aufhellung bewirken und gleichzeitg eine Bleichwirkung vortäuschen, indem sie (unsichtbare) Ultraviolettstrahlung in (sichbares) längerwelliges Licht umwandeln. Das aus dem Sonnenlicht absorbierte ultraviolette Licht wird als schwach bläuliche Fluoreszenz wieder abgestrahlt, also in der Komplementärfarbe der Vergilbung. Diese organischen Leuchtpigmente (Fluoreszenzfarbstoffe) wirken somit wie optischen Transformatoren.

Aus dem Stand der Technik bekannte optische Aufheller enthalten beispielsweise Derivate von 4,4'-Diamino-2,2'-stilbendisulfonsäure (Flavonsäure), 4,4'-Distyrylbiphenylen, Methylumbelliferon, Cumarin, Dihydrochinolinon, 1,3-Diarylpyrazolin, Naphthalsäureimid, über CH=CH-Bindungen verknüpfte Benzoxazol-. Benzisoxazol und Benzimidazol-Systeme oder durch Heterocyclen substituierte Pyren Derivate.

Obwohl mit den bekannten optischen Aufhellern bereits sehr gute Ergebnisse erzielt werden, besteht ein laufender Bedarf nach neuen verbesserten Systemen, da auch die Anforderungen, beispielsweise bezüglich Helligkeit, Echtheit gegenüber Sonnelicht, Waschen, Bügeln, gleichzeitig verwendeten Zusatzstoffen, Umweltverträglichkeit und Kosteneffizienz, ständig steigen.

Es wurde nun überraschend gefunden, daß sich organische Spiroverbindungen, die konjugierte System enthalten, in besonderer Weise zur Verwendung als optische Aufheller eignen.

Spiroverbindungen sind gekennzeichnet durch mindestens ein vierbindiges Spiroatom, das zwei Ringsysteme miteinander verknüpft. Dies wird im Handbook of Chemistry and Physics, 62nd ed. (1981-2), S. C-23 bis 25 ausgeführt.

Ein Gegenstand der Erfindung ist daher die Verwendung eines Spirobifluorenderivates der Formel III, gemäß Anspruch 1

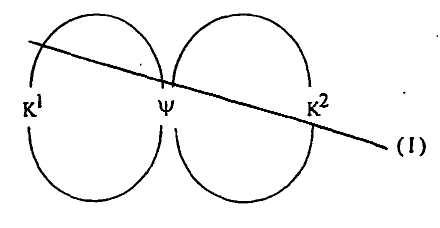

Die erfindungsgemäß verwendeten Verbindungen zeichnen sich gegenüber herkömmlichen optischen Aufhellern durch eine außergewöhnliche Temperaturstabilität aus. Dies zeigt sich z. B. darin, daß das Emissionsmaximum der Verbindungen nach thermischer Belastung nur wenig, in anderen Fällen überhaupt nicht abnimmt und daß bei vielen dieser Verbindungen sogar eine Zunahme des Emissionsmaximums nach thermischer Belastung festgestellt wird. Dadurch wird das Aufhellen von solchen polymeren Materialien möglich, bei denen der Aufheller nur im Schmelzverfahren in das Polymermaterial eingearbeitet werden kann (insbesondere technische polymere Materialien, z. B. Aramid-Fasern. Die dabei erforderlichen hohen Temperaturen führen bei den bisher üblichen Aufhellern zu deren thermischen Abbau. Bei der Herstellung herkömmlicher Fasern kann hier das Naßspinnverfahren durch das lösungsmittelfreie und deshalb aus ökologischen Gründen zu bevorzugende Schmelzverfahren ersetzt werden.

Neben der thermischen Stabilität wird gegenüber den bisher bekannten Aufhellern außerdem eine verbesserte Lichtstabilität, vorzugsweise bei carbocyclischen aromatischen Systemen erreicht.

Aufgrund der höheren thermischen und photochemischen Belastbarkeit im Vergleich zu Aufhellern aus dem Stand der Technik ist das Aufhellen auch auf bisher nicht zugänglichen Anwendungsgebieten möglich. So können diese Verbindungen grundsätzlich auch zum Aufhellen von Geotextilien und, aufgrund geringer Migrationsneigung der Aufhellermoleküle, Verpackungsmaterialien eingesetzt werden.

Die Fluoreszenzquantenausbeute der Spiroverbindungen in Lösung und in der Feststoffmatrix kann über 95 % betragen. Dadurch benötigt man geringere Aufhellerkonzentrationen bei gleichem oder meist verbessertem Aufhelleffekt, was sowohl aus wirtschaftlichen als auch ökologischen Gründen vorteilhaft ist. Aggregationsphänomene, bei herkömmlichen Aufhellern oft ein Problem, treten bei den Spiroverbindungen aufgrund ihrer Struktur nicht auf. Dadurch wird eine günstige und für manche Anwendungsbereiche notwendige molekulardisperse Verteilung erreicht. Die geringe Aggregationsneigung kann darüber hinaus genutzt werden, um sehr hohe Aufhellerkonzentrationen, bis hin zur reinen, vorzugsweise amorphen Verbindung, beispielsweise in Form eines Films, zu erzielen und dennoch eine hohe Fluoreszenzquantenausbeute zu erhalten, d.h. es findet kein Konzentrationsquenching statt.

Weiterhin zeichnen sich diese Verbindungen durch eine hohe Temperaturbeständigkeit im Hinblick auf Farbstabilität und Fluoreszenzquantenausbeute aus. Dies bedeutet, daß sich das Emissionsmaximum im Bereich von 380 bis 750 nm, gemessen bei Raumtemperatur, um nicht mehr als 25 %, relativ zum Ausgangszustand, vermindert, nachdem das Material, aufgetragen in einer Dicke von nicht mehr als 1 µm auf einem Quarzsubstrat, in einer inerten Atmosphäre bei einem Druck von nicht mehr als 1 mbar für 30 min auf 250°C erhitzt wurde.

Vorzugsweise beträgt die Verminderung des Emissionsmaximums nicht mehr als 20 %, besonders bevorzugt 15 %, relativ zum Ausgangszustand vor der thermischen Behandlung.

Die erfindungsgemäß verwendeten Verbindungen der Formel (I) sind dadurch charakterisiert, daß
a) sie eine Fluoreszenzquantenausbeute von ≥ 40 %, vorzugsweise ≥ 50%, besonders bevorzugt ≥ 60 %, im, vorzugsweise amorphen, Festkörper aufweisten und
b) sich das Emissionsmaximum im Bereich von 380 bis 750 nm, gemessen bei Raumtemperatur, um nicht mehr als 25 %, relativ zum Ausgangszustand, vermindert, nachdem das Material, aufgetragen in einer Dicke von nicht mehr als 1 µm auf einem Quarzsubstrat, in einer inerten Atmosphäre bei einem Druck von nicht mehr als 1 mbar für 30 min auf 250°C erhitzt wurde.

Mit den Spiroverbindungen läßt sich durch Variation der Substituenten am Spirobifluorengrundkörper zudem eine Farbnuance einstellen.

Für manche Anwendungen, beispielsweise als effektive, dünne UV-Filter, ist es zudem von Vorteil, daß die erfindungsgemäß eingesetzten Spiroverbindungen amorph präpariert werden können.

Für Filteranwendungen ist es vorteilhaft, daß die Verbindungen durch sehr hohe Extinktionskoeffizienten im UV-Bereich, vorzugsweise zwischen 250 und 380 nm, ausgezeichnet sind und in sehr hohe Aufhellerkonzentrationen, bis hin zum reinen Film (100 %), als amorphe dünne Filme (beispielsweise durch Spin-Coating oder Sublimation) präparierbar sind.

Der Begriff amorph dient zur Zustandsbeschreibung von Festkörpern, deren molekulare Bausteine nicht in Kristallgittern, sondern regellos angeordnet sind. Anders als in einem Kristall, bei dem zwischen den Atomen eine Nahordnung (d.h. konstante Abstände zu nächsten Nachbaratomen) und eine Femordnung (regelmäßiges Wiederholen eines Basisgitters) existiert, liegt im amorphen Zustand nur eine Nahordnung vor. Der amorphe Stoff besitzt keine physikalisch ausgezeichnete Richtung; er ist isotrop. Alle amorphen Stoffe streben unterschiedlich stark den energetisch günstigeren kristallinen Zustand an. Bei Beugung von Röntgen-, Elektronen- und Neutronenstrahlen zeigen sich beim amorphen Festkörper keine scharfen Interferenz-Ringe, wie bei einem Kristall, sondern nur diffuse Interferenzringe bei kleinen Beugungswinkeln (Halos).

Der amorphe Zustand ist somit klar vom kristallinen, flüssigen oder auch flüssigkristallinen Zustand unterschieden.

Die erfindungsgemäßen Spiroverbindungen sind im Vergleich zu vielen bekannten Systemen auch gut löslich, besonders in polaren Solventien, insbesondere Dichlormethan und Chloroform (> 30 g/l), so daß u. a. auch Spin-Coating und Filmbildung möglich ist.

Eine Verarbeitbarkeit aus wäßrigen Systemen wird durch Substitution der Spiroverbindungen mit stark polaren Gruppierungen, wie Carbonsäure-, Carboxylat-, Sulfonsäure-, Sulfonat- und quartäre Ammoniumgruppierungen, erreicht.

Die Emissionseigenschaften der erfindungsgemäß eingesetzten Verbindungen können durch die Wahl geeigneter Substituenten über den ganzen Bereich des sichtbaren Spektrums eingestellt werden. Darüber hinaus erlaubt die kovalent gebundene Anordnung der zwei Teile der Spiroverbindung einen molekularen Aufbau in der Weise, daß in beiden Hälften des Moleküls unabhängig voneinander bestimmte Eigenschaften eingestellt werden können, z.B. eine Ausdehnung des Absorptionsbereichs ins langwellige UV.

Bevorzugt werden Verbindungen gemäß Anspruch 2 und 3 verwendet.

Ganz besonders bevorzugte Spiroverbindungen sind solche der Formel (IV), 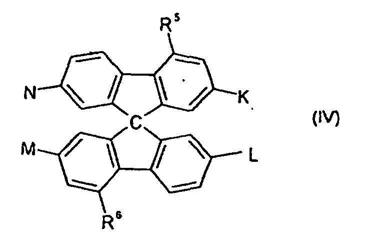 wobei die Symbole folgende Bedeutungen haben:
K, L, M, N, R⁵, R⁶ sind gleich oder verschieden eine der Gruppen G1 bis G11: 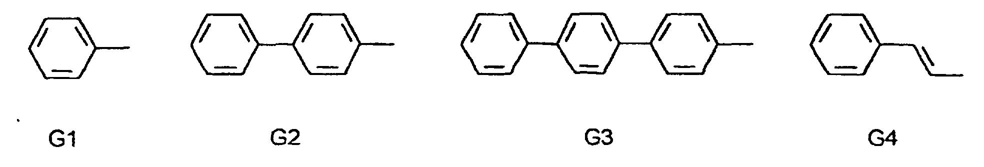 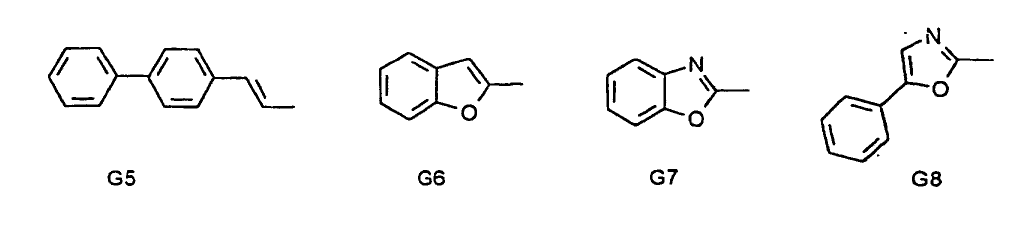 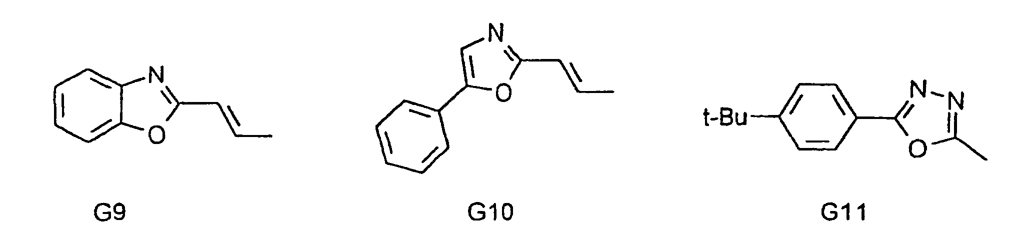 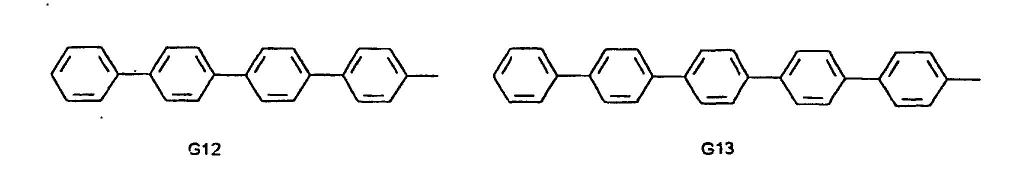
   - und R⁵, R⁶: können auch gleich oder verschieden Wasserstoff oder eine lineare oder verzweigte Alkyl-, Alkyloxy- oder Estergruppe mit 1 bis 22 C-Atomen, -CN oder -NO₂ bedeuten.

Insbesondere bevorzugte Spiroverbindungen der Formel (IV) sind die in der Tabelle 1 aufgeführten Verbindungen, bei denen die Abkürzungen G1 bis G11 die in der Formel (IV) angegebenen Bedeutungen haben.

**Tabelle 1:**

| Spiroverbindungen der Formel (IV) R⁵= R⁶ = Wasserstoff | | | | |
|---|---|---|---|---|
| Verbindung | K | L | M | N |
| Spiro-1 | G1 | G1 | G3 | G3 |
| Spiro-2 | G1 | G1 | G4 | G4 |
| Spiro-3 | G1 | G1 | G5 | G5 |
| Spiro-4 | G1 | G1 | G6 | G6 |
| Spiro-5 | G1 | G1 | G7 | G7 |
| Spiro-6 | G1 | G1 | G8 | G8 |
| Spiro-7 | G1 | G1 | G9 | G9 |
| Spiro-8 | G1 | G1 | G10 | G10 |
| Spiro-9 | G1 | G1 | G11 | G11 |
| Spiro-10 | G2 | G2 | G2 | G2 |
| Spiro-11 | G2 | G2 | G3 | G3 |
| Spiro-12 | G12 | G12 | G12 | G12 |
| Spiro-13 | G13 | G13 | G13 | G13 |

Bevorzugt sind auch Spiroverbindungen der Formel (V), 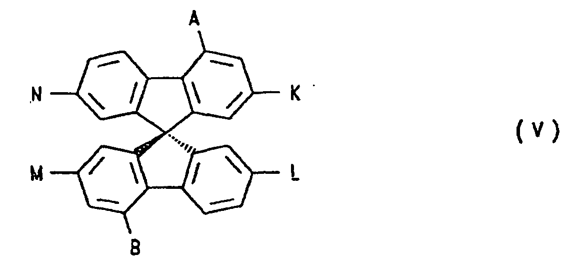 wobei A, B, K, L, M, N gleich oder verschieden sind und die Bedeutungen haben, wie sie für K, L, M und N bei der Formel (III) angegeben sind.

Bevorzugt sind Verbindungen der Formel (V) bei denen K, L, M, N, A und B die bereits zuvor aufgeführten Gruppen G 1 bis G 13 darstellen.

Insbesondere bevorzugte Spiroverbindungen der Formel (V) sind
2,2',4,4',7,7'- Hexakis(biphenylyl)-9,9'-spirobifluoren,
2,2',4,4',7,7'- Hexakis(terphenylyl)-9,9'-spirobifluoren
2,2',4,4',7,7'-Hexakis(quaterphenylyl)-9,9'-spirobifluoren
2,2',4,4',7,7'-Hexakis(pentaphenylyl)-9,9'-spirobifluoren

Die Herstellung der erfindungsgemäß verwendeten Spiroverbindungen erfolgt nach an sich literaturbekannten Methoden, wie sie in Standardwerken zur Organischen Synthese, z.B. Houben-Weyl, Methoden der Organischen Chemie, Georg-Thieme-Verlag, Stuttgart und in den entsprechenden. Bänden der Serie "The Chemistry of Heterocyclic Compounds" von A. Weissberger und E. C. Taylor (Herausgeber) beschrieben werden.

Die Herstellung erfolgt dabei unter Reaktionsbedingungen, die für die genannten Umsetzungen bekannt und geeignet sind. Dabei kann auch von an sich bekannten, hier nicht näher erwähnten Varianten Gebrauch gemacht werden.

### Carbonspiroverbindungen (ψ = C)

Verbindungen der Formel (III) werden beispielsweise ausgehend vom 9,9'-Spirobifluoren erhalten, dessen Synthese z.B. von R. G. Clarkson, M. Gomberg, J.Am.Chem.Soc. 52 (1930) 2881, beschrieben ist.

Die Herstellung von Verbindungen der Formel (IIIa) kann beispielsweise ausgehend von einer Tetrahalogenierung in den Positionen 2,2',7,7' des 9,9'-Spirobifluorens und anschließender Substitutionsreaktion erfolgen (siehe z.B.
US 5,026,894) oder über eine Tetraacetylierung der Positionen 2,2',7,7' des 9,9'-Spirobifluorens mit anschließender C-C-Verknüpfung nach Umwandlung der Acetylgruppen in Aldehydgruppen oder Heterocyclenaufbau nach Umwandlung der Acetylgruppen in Carbonsäuregruppen erfolgen.

Die Herstellung von Verbindungen der Formel (IIIb) kann beispielsweise analog zu denen der Formel IIIa erfolgen, wobei die stöchiometrischen Verhältnisse bei der Umsetzung so gewählt werden, daß die Positionen 2,2' bzw. 7,7' funktionalisiert werden (siehe z.B. J. H. Weisburger, E. K. Weisburger,
F. E. Ray, J. Am. Chem. Soc. 72 (1959) 4253; F. K. Sutcliffe, H. M. Shahidi, D. Paterson, J. Soc. Dyers Colour 94 (1978) 306 und G. Haas, V. Prelog, Helv. Chim. Acta 52 (1969) 1202).

Die Herstellung von Verbindungen der Formel (IIIc) kann beispielsweise über eine Dibromierung in 2,2'Stellung und anschließender Diacetylierung in 7,7' Stellung des 9,9'-Spirobifluorens und anschließende Umsetzung analog zu der der Verbindungen IIIa erfolgen.

Verbindungen der Formeln (IIIe)-(IIIg) sind beispielsweise durch Wahl geeignet substituierter Ausgangsverbindungen beim Aufbau des Spirobifluorens herstellbar, z. B. kann 2,7-Dibromspirobifluoren aus 2,7-Dibromfluorenon und 2,7-Dicarbethoxy-9,9-spirobifluoren durch Einsatz von 2,7-Dicarbethoxyfluorenon aufgebaut werden. Die freien 2,'7'-Positionen des Spirobifluorens können dann unabhängig weiter substituiert werden.

Für die Synthese der Gruppen K, L, M, N, sei beispielsweise verwiesen auf
DE-A 23 44 732, 24 50 088, 24 29 093, 25 02 904, 26 36 684, 27 01 591 und 27 52 975 für Verbindungen mit 1,4-Phenylen-Gruppen;
DE-A 26 41 724 für Verbindungen mit Pyrimidin-2,5-diyl-Gruppen;
DE-A 40 26 223 und EP-A 03 91 203 für Verbindungen mit Pyridin-2,5-diyl-Gruppen; DE-A 32 31 462 für Verbindungen mit Pyridazin-3,6-diyl-Gruppen; N. Miyaura, T. Yanagi und A. Suzuki in Synthetic Communications 11 (1981) 513 bis 519, DE-A-3 930 663, M. J. Sharp, W. Cheng, V. Snieckus in Tetrahedron Letters 28 (1987), 5093; G. W. Gray in J. Chem. Soc. Perkin Trans II (1989) 2041 und Mol. Cryst. Liq. Cryst. 172 (1989) 165, Mol. Cryst. Liq. Cryst. 204 (1991) 43 und 91; EP-A 0 449 015; WO 89/12039; WO 89/03821; EP-A 0 354 434 für die direkte Verknüpfung von Aromaten und Heteroaromaten;

Die Herstellung disubstituierter Pyridine, disubstituierter Pyrazine, disubstituierter Pyrimidine und disubstituierter Pyridazine findet sich beispielsweise in den entsprechenden Bänden der Serie "The Chemistry of Heterocyclic Compounds" von A. Weissberger und E. C. Taylor (Herausgeber).

Die Herstellung erfolgt unter Reaktionsbedingungen, die für die genannten Umsetzungen bekannt und geeignet sind. Dabei kann auch von an sich bekannten, hier nicht näher erwähnten Varianten Gebrauch gemacht werden.

Die Herstellung von Verbindungen der Formel (IIIa) kann beispielsweise ausgehend von einer Tetrahalogenierung in den Positionen 2,2',7,7' des 9,9'-Spirobi-9-fluorens und anschließender Substitutionsreaktion erfolgen die von analogen C-Spiroverbindungen bekannt sind (siehe z.B. US-A-5,026,894). Dies kann beispielsweise über die entsprechenden Cyanoverbindungen zu Aldehyd- oder Carbonsäurefunktionalität führen, welche, z. B. zum Aufbau von Heterocyclen verwendet werden.

Die Herstellung von Verbindungen der Formel (Itlb) kann beispielsweise analog zu denen der Formel (lila) erfolgen, wobei die stöchiometrischen Verhältnisse bei der Umsetzung so gewählt werden, daß die Positionen 2,2' bzw. 7,7' funktionalisiert werden (siehe z.B. J. H. Weisburger, E. K. Weisburger,
F. E. Ray, J. Am. Chem. Soc. 1959, 72, 4253; F. K. Sutcliffe, H. M. Shahidi,
D. Paterson, J. Soc. Dyers Colour 1978, 94, 306 und G. Haas, V. Prelog, Helv. Chim. Acta 1969, 52, 1202).

Die Herstellung von Verbindungen der Formel (IIIc) und (IIId) kann beispielsweise über eine Dibromierung in 7,7'-Stellung des analog zu (IIIa) synthetisierten 2,2'-Dicyano- in 9,9'-Spirobi-9-fluorens und anschließende Umsetzungen analog zu denen bei Verbindungen (IIIa) erfolgen.

Darüber hinaus sind die dem Fachmann geläufigen Synthesesequenzen, wie Nitrierung, Reduzierung, Diazotierung und Sandmeyer-Reaktion, einzusetzen.
Für die Synthese der Gruppen K, L, M, Q sei beispielsweise verwiesen auf DE-A 23 44 732, 24 50 088, 24 29 093, 25 02 904, 26 36 684, 27 01 591 und 27 52 975 für Verbindungen mit 1,4-Phenylen-Gruppen;
DE-A 26 41 724 für Verbindungen mit Pyrimidin-2,5-diyl-Gruppen;
DE-A 40 26 223 und EP-A 0 391 203 für Verbindungen mit Pyridin-2,5-diyl-Gruppen;
DE-A 32 31 462 für Verbindungen mit Pyridazin-3,6-diyl-Gruppen; N. Miyaura, T. Yanagi und A. Suzuki in Synthetic Communications 11 (1981) 513 bis 519, DE-A-39 30 663, M. J. Sharp, W. Cheng, V. Snieckus in Tetrahedron Letters 1987, 28, 5093; G. W. Gray in J. Chem. Soc. Perkin Trans II, 1989, 2041 und Mol. Cryst. Liq. Cryst. 1989, 172, 165, Mol. Cryst. Liq. Cryst. 1991, 204, 43 und 91; EP-A 0 449 015; WO 89/12039; WO 89/03821; EP-A 0 354 434 für die direkte Verknüpfung von Aromaten und Heteroaromaten;

Die Herstellung disubstituierter Pyridine, disubstituierter Pyrazine, disubstituierter Pyrimidine und disubstituierter Pyridazine findet sich beispielsweise in den entsprechenden Bänden der Serie "The Chemistry of Heterocyclic Compounds" von A. Weissberger und E. C. Taylor (Herausgeber).

Die vorstehend beschriebenen Spiroverbindungen zeigen in gelöstem, feinverteiltem oder festem Zustand eine ungewöhnlich ausgeprägte Fluoreszenz. Sie werden zum optischen Aufhellen der verschiedensten synthetischen, halbsynthetischen oder natürlichen organischen Materialien oder Substanzen, welche organische Materialien enthalten, verwendet.

Als geeignet zur optischen Aufhellung seien beispielsweise die folgenden Gruppen von Materialien genannt:
I. Synthetische, hochmolekulare Materialien:
   a) Polymerisationsprodukte auf Basis von mindestens eine polymerisierbare Kohlenstoff-Kohlenstoff-Doppelbindung enthaltenden Verbindungen, d. h. deren Homo- oder Copolymerisate sowie deren Nachbehandlungsprodukte, wie beispielsweise Vernetzungs-, Propfungs- oder Abbauprodukte, Polymerisat-Verschnitte oder durch Modifizierung reaktionsfähiger Gruppen erhaltene Produkte, beispielsweise Polymerisate auf Basis ungesättigter Carbonsäuren oder deren Derivaten, insbesondere Acrylverbindungen (wie Acrylestern, Acrylsäure, Acrylnitril, Acrylamide und deren Derivaten oder deren Methacryl-Analoga), Polymerisate von Olefinen (wie z. B. Ethylen, Propylen, Styrole oder Diene, ferner ABS-Polymerisate), Polymerisate auf Basis von Vinyl- und Vinyliden-Verbindungen (wie Vinylchlorid und Vinylalkohol),
   b) Polymerisationsprodukte, die durch Ringöffnung erhältlich sind, z. B. Polyamide vom Polycaprolactam-Typ, ferner Polymere, die sowohl über Polyaddition als auch Polykondensation erhältlich sind, wie Polyether oder Polyacetale,
   c) Polykondensationsprodukte oder Vorkondensate auf Basis bi- oder polyfunktioneller Verbindungen mit kondensätionsfähigen Gruppen, deren Homo- und Mischkondensationsprodukte, sowie Produkte der Nachbehandlung, wie beispielsweise Polyester, insbesondere gesättigte (z. B. Ethylenglykolterephthalsäure-Polyester) oder ungesättigte (z. B. Maleinsäüre-Dialkohol-Polykondensate sowie deren Vernetzungsprodukte mit anpolymerisierbaren Vinylmonomeren), unverzweigte (auch auf Basis höherwertiger Alkohole, wie Alkydharze) Polyester, Polyamide (z. B. Hexamethylendiamin-adipat), Maleinatharze, Melaminharze, deren Vorkondensate und Analoga, Polycarbonate, Silikone,
   d) Polyadditionsprodukte, wie Polyurethane (vernetzt und unvernetzt) und Epoxidharze.
II. Halbsynthetische Materalien, z. B. Celluloseester verschiedener Veresterungsgrade (z. B. sogenanntes 2 1/2-Acetat, Triacetat) oder Celluloseether, regenerierte Cellulose (Viskose, Kupferammoniak-Cellulose) oder deren Nachbehandlungsprodukte, Casein-Kunststoffe.
III. Natürliche organische Materialien animalischen oder vegetabilischen Ursprungs, beispielsweise auf Basis von Cellulose oder Proteinen wie Baumwolle, Wolle, Leinen, Seide, natürliche "Lackharze", Stärke, Casein.

Bevorzugte organische Materialien sind solche aus Polyester, Polyethylen, Polypropylen, Polystyrol, Polyacryl, Polyamid, Polymethan, Polyvinylchlorid, Acetylcellulose, Polyethylenterephthalat und technische Kunststoffe, wie beispielsweise Aramidfasem.

Die optisch aufzuhellenden organischen Materialien können den verschiedenartigsten Verarbeitungszuständen (Rohstoffe, Halbfabrikate oder Fertigfabrikate) angehören. Sie können andererseits in Form der verschiedenartigsten geformten Gebilde vorliegen, beispielsweise als vorwiegend dreidimensional ausgedehnte Körper, wie Platten, Profile, Spritzgußformlinge, verschiedenartige Werkstücke, Schnitzel, Granulate oder Schaumstoffe, ferner als vorwiegend zweidimensional ausgebildete Körper, wie Filme, Folien, Lacke, Überzüge, Imprägnierungen und Beschichtungen oder als vorwiegend eindimensional ausgebildete Körper, wie Fäden, Fasern, Flocken, Drähte. Diese Materialien können andererseits auch in ungeformtem Zustand in den verschiedenartigsten homogenen oder inhomogenen Verteilungsformen, z. B. als Pulver, Lösungen, Emulsionen, Dispersionen, Latices, Pasten oder Wachse vorliegen.

Den erfindungsgemäß anzuwendenden Verbindungen kommt vor allem Bedeutung als optische Aufheller für Polymere, insbesondere transparente Polymere zu.

Fasermaterialien können beispielsweise als endlose Fäden (verstreckt oder unverstreckt), Stapelfasern, Flocken, Strangware, textile Fäden, Garne, Zwirne, Fasernvliese, Filze, Watten, Beflockungs-Gebilde oder als textile Gewebe oder textile Verbundstoffe, Gewirke sowie als Papiere, Pappen oder Papiermassen vorliegen.

Sofern Fasern, welche als Stapelfasern oder Endlosfäden, in Form von Strangen, Geweben, Gewirken, Vliesen, beflockten Substraten oder Verbundstoffen vorliegen können, erfindungsgemäß optisch aufzuhellen sind, geschieht dies mit Vorteil in wäßrigem Medium, worin die betreffenden Verbindungen in feinverteilter Form (Suspensionen, sogenannte Mikrodispersionen, gegebenenfalls Lösungen) vorliegen. Gegebenenfalls können bei der Behandlung Dispergier-, Stabilisier-, Netz- und weitere Hilfsmittel zugesetzt werden.

In Abhängigkeit vom verwendeten Aufheller-Verbindungstyp kann es sich als vorteilhaft erweisen, in neutralen oder alkalischem oder saurem Bad zu arbeiten. Die Behandlung wird üblicherweise bei Temperaturen von 20 °C bis 140 °C, beispielsweise bei Siedetemperatur des Bades oder in deren Nähe, durchgeführt. Wegen der überragenden Temperaturstabilität der Spiro-Verbindungen können auch Badtemperaturen bis zu 400°C gewählt werden. Für die erfindungsgemäße Veredelung textiler Substrate kommen auch Lösungen oder Emulsionen in organischen Lösungsmitteln in Betracht, wie dies in der Färbereipraxis in der sogenannten Lösungsmittelfärberei (Foulard-Thermofixier-Applikation, Ausziehfärbeverfahren in Färbemaschinen) praktiziert wird.

Die optischen Aufheller können erfindungsgemäß beispielsweise auch zur Aufhebung von Papiermassen, unter anderem auch in Gegenwart von beispielsweise kationischen Retentionsmitteln und anderen Zusatzstoffen eingesetzt werden.

Die Spiro-Verbindungen können ferner den Materialien vor oder während der Verformung zugesetzt bzw. eingearbeitet werden. So kann man sie beispielsweise bei der Herstellung von Filmen, Folien (z. B. Einwalzen in Polyvinylchlorid in der Hitze) oder von Formkörpern der Pressmasse oder Spritzgußmasse beifügen. Der besondere Vorteil der Spiroverbindungen liegt dabei darin, daß auch höhere Verarbeitungstemperaturen (bis zu 400°C) als bisher üblich angewendet werden können. Dadurch ist insbesondere das Aufhellen von technischen Kunststoffen, die höhere Verarbeitungstemperaturen erfordern, möglich geworden (z. B. Aramid-Fasern).

Sofern die Formgebung der Materialien durch Spinnverfahren oder über Spinnmassen erfolgt, können die optischen Aufheller nach folgenden Verfahren appliziert werden:
- Zugabe zu den Ausgangssubstanzen (z. B. Monomeren) oder Zwischenprodukten (z. B. Vorkondensaten, Praepolymeren), d. h. vor oder während der Polymerisation, Polykondensation oder Polyaddition,
- Aufpudern auf Polymerisationsschnitzeln oder Granulate für Spinnmassen,
- Badfärbung von Polymerisationsschnitzeln oder Granulaten für Spinnmassen,
- Dosierte Zugabe zu Spinnschmelzen oder Spinnlösungen. Durch die absolute Beständigkeit gegenüber Natriumrhodanid, ist die Einarbeitung in Spinnmassen für die Herstellung von Polyacrylnitrilfasern im sogenannten Naßspinnverfahren auf Basis rhodanidhaltiger Spinn- und Fällungsbäder besonders von Interesse
- Applikation auf Spinnkabel vor dem Verstrecken.

Die optischen Aufheller können gemäß vorliegender Erfindung beispielsweise in folgenden Anwendungsformen eingesetzt werden:
a) Mischungen bzw. molekulare Dispersionen mit Polymeren, vorzugsweise transparenten Polymeren, wie Acrylaten, Methacrylaten, Carbonaten, Polyestern (z.B. PET), Polyethern oder Epoxyharzen,
b) Mischungen mit Farbstoffen (Nuancierung) oder Pigmenten (Farb- oder insbesondere auch Weißpigmente) oder als Zusatz zu Färbebädern, Druck-, Ätz- oder Reservepasten. Ferner auch zur Nachbehandlung von Färbungen, Drucken oder Aetzdrucken,
c) in Mischungen mit sogenannten Carriern, Netzmitteln, Weichmachern, Quellmitteln, Antioxydantien, Antifungiziden und Antibakteriziden, Lichtschutzmitteln, Hitzestabilisatoren, chemischen Bleichmitteln (beispielsweise Chlorit-Bleichen, Wasserstoffperoxid- bzw. Peroxidische Bleichen, Percarbonsäure-Bleichen, Bleichbäder-Zusätze); der Zusatz reduzierend wirkender Verbindungen, beispielsweise Schwefelverbindungen, ist bei Spirobifluorenverbindungstypen bedeutsam. Besonders bevorzugte Handelsformen sind dabei konzentrierte, wäßrige Lösungen. Die reduzierend wirkenden Schwefelverbindungen können sowohl organischer wie anorganischer Natur sein und sind vorzugsweise wasserlöslich. So eignen sich z. B. Dithionite, Pyrosulfite, Sulfite, Sulfide, Thiosulfate und Thiocyanate (z. B. Kaliumrhodanid) in Form ihrer Salze (z. B. Alkali-, Erdalkali- oder Ammoniumsalze) als wäßrige Lösungen, in fester Form oder soweit bekannt auch in Form der freien Säuren oder deren Anhydride, wie Schwefeldioxid. Als Vertreter organischer Verbindungen seien Mercaptane, wie Thioglykolsäure, Mercaptoethanol, 4-Hydroxy-2-mercapto-3,3'-dithiodipropionsäure, Sulfinate, wie Natriumformaldehydsulfoxylat oder Formamidinsulfinsäure sowie Thioharnstoff genannt. Besonders bevorzugt ist Na-Dithionit.
   Die Menge der Schwefelverbindung beträgt im allgemeinen 0,05 - 10 Mol-% bezogen auf den Aufheller, vorzugsweise 0,5 - 5 Mol-%,
d) in Mischung mit Vernetzern, Appreturmitteln (z. B. Stärke oder synthetischen Appreturen) sowie in Kombination mit den verschiedensten Textilveredlungsverfahren, insbesondere Kunstharzausrüstungen
   (z. B. Knitterfest-Ausrüstungen, wie "wash- and -wear", "permanent-press", "no-iron"), ferner Antistatisch-Ausrüstungen oder antimikrobiellen Ausrüstungen,
e) Einarbeiten der optischen Aufheller in polymere Trägermaterialien (Polymerisations-, Polykondensations- oder Polyadditionsprodukte) in gelöster oder dispergierter Form für Anwendungen in Beschichtungs-, Imprägnier- oder Bindemitteln (Lösungen, Dispersionen, Emulsionen) für Textilien, Vliese, Papier, Leder,
f) als Zusätze zu sogenannten "master batches",
g) als Zusätze zu den verschiedensten industriellen Produkten, um dieselben marktfähiger zu machen (z. B. Aspektverbesserung von Seifen, Waschmitteln, Pigmenten, PET-Flaschen, Aramid-Fasern und Nähfäden für Sportartikel, Schuhe, hochreißfeste Seile, schußsichere Westen, Dachpappen, deponierbare Folien),
h) in Kombination mit anderen, optisch aufhellend wirkenden Substanzen, insbesondere Mischungen von optischen Ausfhellern bestehend aus 1 bis 60 Gew.-% eines Aufhellers aus der Reihe der Bisbenzoxazolyl-substituierten Spirobifluorene und 99 bis 40 Gew.-% eines oder mehrerer handelsüblicher Aufheller, wie beispielsweise Aufheller aus der Reihe der Coumarin-, Stilben- oder Pyrenaufheller. Da einige der Spiroverbindungen Aufhelleffekte mit grüner Nuance bewirken, ist es besonders vorteilhaft, sie gemeinsam mit Aufhellern zu verwenden, die rotstichige Aufhelleffekte zeigen (z. B. Verbindungen aus der Klasse der 2-Stilbenylnaphthotriazole. Bevorzugt sind auch Mischungen der Spiroverbindungen, wobei das optimale Mischungsverhältnis im Einzelfall von der Art der jeweiligen Spiroverbindungen abhängt und sich durch einfache Vorversuche unschwer ermitteln läßt.
   In Einzelfällen können mit solchen Mischungen auch unerwartete synergistische Effekte in Bezug auf den Weißgrad und die Brillianz der Aufhellungen erzielt werden,
i) in Spinnbadpräparationen, d. h. als Zusätze zu Spinnbädern, wie sie zur Gleitfähigkeitsverbesserung für die Weiterverarbeitung von Synthese-Fasern verwendet werden, oder aus einem speziellen Bad vor der Verstreckung der Faser,
j) als Scintillatoren, für verschiedene Zwecke photographischer Art, z. B. für elektrophotographische Reproduktionen oder Supersensibilisierung, zur optischen Aufhellung photographischer Schichten.

Aus verschiedenen Gründen ist es oft zweckmäßig, die Aufheller nicht als solche, d. h. rein, einzusetzen, sondern vermischt mit den verschiedensten Hilfs- und Coupiermitteln, wie wasserfreiem Natriumsulfat, Natriumsulfat-decahydrat, Natriumchlorid, Natriumcarbonat, Alkalimetallphosphaten, wie Natrium- oder Kaliumtripolyphosphate oder Alkalimetallsilikaten.

In gewissen Fällen werden die Aufheller durch eine Nachbehandlung zur vollen Wirkung gebracht. Diese kann beispielsweise eine chemische (z. Bsp. SäureBehandlung), eine thermische (z. Bsp. Hitze) oder eine kombinierte chemisch/thermische Behandlung darstellen. So verfährt man beispielsweise bei der optischen Aufhellung einer Reihe von Fasersubstanzen, z. B. von Polyesterfasern, mit den Aufhellern zweckmäßig in der Weise, daß man diese Fasern mit wäßrigen Dispersionen (gegebenenfalls auch Lösungen) der Aufhellmittel bei Temperaturen zwischen 80 °C und Raumtemperatur imprägniert und einer trockenen Wärmebehandlung bei Temperaturen über 100 °C unterwirft, wobei es sich im allgemeinen empfiehlt, das Fasermaterial vorher noch bei mäßig erhöhter Temperatur, z. Bsp. bei mindestens 60 °C bis etwa 180°C zu trocknen. Die Wärmebehandlung in trockenem Zustande erfolgt dann vorteilhaft bei Temperaturen zwischen 120 und 225 °C, beispielsweise durch Erwärmen in einer Trockenkammer, durch Bügeln im angegebenen Temperaturintervall oder durch Behandeln mit trockenem, überhitztem Wasserdampf. Die Trocknung und trockene Wärmebehandlung können auch unmittelbar nacheinander ausgeführt oder in einen einzigen Arbeitsgang zusammengeführt werden.

Die Menge der erfindungsgemäß zu verwendenden optischen Aufheller, bezogen auf das aufzuhellende Material, kann in weiten Grenzen schwanken. Schon mit außergewöhnlich geringen Mengen, in gewissen Fällen z. B. solchen von 10⁻⁶ Gewichtsprozent, kann ein deutlicher und haltbarer Effekt erzielt werden. Es können aber auch Mengen bis gegebenenfalls etwa 3 Gewichtsprozent genommen werden. Für viele praktische Belange sind vorzugsweise Menge zwischen 0,00001 und 0,5 Gewichtsprozent von Interesse. Für manche Anwendungen, beispielsweise als Transformationsmaterial, insbesondere in Schichtform, für UV-Licht zur Erhöhung des Wirkungsgrades in Solarzellen, kann die Menge des erfindungsgemäßen Aufhellers auch bedeutend höher (3 bis 90 Gew.-%, vorzugsweise 10 bis 75 Gew.-%, besonders bevorzugt 10 bis 50 Gew.-%) sein. Der oder den zusätzlich verwendeten Komponenten, vorzugsweise transparenten Polymeren, kommt in diesen Fällen üblicherweise die Rolle eines Bindemittels zu.

Es ist in diesem Zusammenhang von besonderem Vorteil, daß sich zumindest die Spiroverbindungen der Formel (I) in jedem Verhältnis mit bekannten Polymeren mischen lassen.

Die Spiro-Verbindungen eignen sich auch als Zusätze für Waschbäder oder bei Gewerbe- und Haushaltswaschmitteln, insbesondere auch bei konzentrierten, flüssigen oder festen Waschmitteln. Zu Waschbädern werden die Aufheller zweckmäßig in Form ihrer Lösungen in Wasser oder organischen Lösungsmitteln oder auch in feiner Verteilung als wäßrige Dispersionen zugegeben. Bei Haushalt- oder gewerblichen Waschmitteln werden sie vorteilhaft in irgendeiner Phase des Herstellprozeßes der Waschmittel, z. B. der sogenannten "slurry", vor dem Zerstäuben zugesetzt. Die Zugabe kann sowohl in Form einer Lösung oder Dispersion in Wasser oder anderen Lösungsmitteln als auch ohne Hilfsstoffe als trockenes Aufhellerpulver erfolgen. Man kann die Aufheller beispielsweise mit den waschaktiven Substanzen vermischen, verkneten oder vermahlen und so dem fertigen Waschpulver zumischen. Sie können jedoch auch gelöst oder vordispergiert auf das fertige Waschmittel aufgesprüht werden.

Als Waschmittel kommen die bekannten Mischungen von Waschaktivsubstanzen, wie beispielsweise Seife in Form von Schnitzeln und Pulver, Synthetika, lösliche Salze von Sulfonsäurehalbestern höherer Fettalkohole, höher und/oder mehrfach alkylsubstituierte Arylsulfonsäuren, Sulfocarbonsäureester mittlerer bis höherer Alkohole, Fettsäureacylaminoalkyl- oder -aminoaryl-glycerinsulfonate, Phosphonsäureester von Fettalkoholen usw., in Frage, sowie übliche Tenside, beispielsweise die wasserlöslichen Produkte, die aus der Addition eines Alkylenoxides bzw. einer äquivalenten Verbindung mit einem reaktiven Wasserstoffatom einer hydrophoben Verbindung erhalten werden. Die hydrophoben organischen Produkte können Heterocyclen und besonders Aliphaten oder Aromaten sein. Bevorzugt sind höhere aliphatische Alkohole und Alkylphenole, wobei aber auch andere, z. B. Carbonsäuren, Carboxamide, Mercaptane und Sulfamide, verwendet werden können. Bevorzugte nichtionogene Verbindungen sind die Additionsprodukte von Ethylenoxid mit höherem aliphatischen Alkoholen mit 6 bis 50 und mehr C-Atomen. Die Menge Ethylenoxid kann sich in weiteren Grenzen bewegen, aber im allgemeinen werden zumindest 5 Mol Ethylenoxid pro Mol hydrophober Substanz gebraucht. Anstelle von Ethylenoxid können ganz oder teilweise andere niedere Alkylenoxide, wie Propylenoxid und Butylenoxid, verwendet werden.

Als weitere nichtionogene Tenside kommen in beispielsweise Betracht:
a) Polyoxyalkylenester organischer Säuren, wie höhere Fettsäuren, Harzsäuren, Talgölsäuren und Säuren der Oxidationsprodukte des Erdöls, deren Ester in der Regel im Säureteil 10 bis 22 C-Atome aufweisen und ca. 12 bis ca. 30 Mole Ethylenoxid oder dessen Äquivalent enthalten.
b) Alkylenoxidaddukte höherer Fettsäureamide, wobei der Fettsäureanteil in der Regel 8 bis 22 C-Atome aufweist und mit 10 bis 50 Mol Ethylenoxid kondensiert ist. Die entsprechenden Carboxamide und Sulfamide können ebenfalls verwendet werden.

Bei der Herstellung von konzentrierten Waschmitteln werden als nichtionogene Tenside vorzugsweise oxalkylierte höhere aliphatische Alkohole verwendet, wobei die Fettalkohole mindestens 6 und vorzugsweise mindestens 8 C-Atome aufweisen. Bevorzugte Alkohole sind Lauryl-, Myristyl-, Cetyl-, Stearyl- und Oleylalkohol, welche mit mindestens 6 Mol Ethylenoxid kondensiert werden. Als typisches nichtionogenes Produkt sei das Additionsprodukt von einem aliphatischen Alkohol mit 12 - 13 C-Atomen mit ca. 6,5 Mol Ethylenoxid erwähnt. Die entsprechenden Alkylmercaptane sind, nach Kondensation mit Ethylenoxid, ebenfalls als nichtionogene Tenside verwendbar.

Die alkoxylierten höheren Alkohole sind besonders für Haushaltswaschmittel geeignet, da sie leicht biologisch abbaubar sind und gut mit kationischen Tensiden und Textilweichmachern und den übrigen Zusätzen verträglich sind.

Als Aufbaustoffe, sogenannte "Builders", kommen z. B. Alkalipoly- und - polymetaphosphate, Alkalipyrophosphate, Alkalisalze der Carboxymethylcellulose und andere "Soilredepositionsinhibitoren'', ferner Alkalisilikate, Alkalicarbonate, Alkaliborate, Alkaliperborat, Nitrilotriessigsäure, Ethylendiaminotetraessigsäure, Schaumstabilisatoren, wie Alkanolamide höherer Fettsäuren, in Betracht. Ferner können in den Waschmitteln beispielsweise enthalten sein: antistatische Mittel, rückfettende Hautschutzmittel, wie Lanolin, Enzyme, Antimikrobika, Parfüme, Farbstoffe und kationische Textilweichmacher.

Als kationische Textilweichmacher kommen vor allem quaternäre Derivate des Ammoniaks und/oder des Imidazolins mit 2 langkettigen, aliphatischen gesättigten oder ungesättigten Resten in Betracht.

Beispiele für quaternäre Ammonium-Weichmacher sind:
Tallyltrimethylammoniumchlorid, Ditallyldimethylammoniumchlorid;
Ditallyldimethylammoniumsulfat, Dihexadecyldimethylammoniumchlorid;
Dioctadecyldimethylammoniumchlorid, Dieicosyldimethylammoniumchlorid,
Didocosyldimethylammoniumchlorid, Dihexadecyldiethylammoniumchlorid,
Dihexdecylmethylammoniumacetat, Ditallyldipropylammoniumphosphat,
Ditallyldimethylammoniumnitrat, Dicocoyldimethammoniumchlorid.
1-Methyl-1-stearylamidoethyl-2-heptadecyl-imidazoliniummethosulfat,
1-Methyl-1-palmitoylamidoethyl-2-octadecyl-imidazoliniumchlorid,
2-Tallyl-1-methyl-1-talloylamidoethyl-imidazoliniummethosulfat.

Weiter sind als Textilweichmacher beispielsweise geeignet:
1-Methyl-1-oleylamidoethyl-2-octadecyl-imidazoliniumchlorid,
1-Methyl-1-talloylamidoethyl-2-tallyl-imidazoliniumchlorid,
Di-tallyl-dimethylammoniumchlorid,
1-Methyl-1-oleylamidoethyl-2-oleyl-imidazolinium-methosulfat,
1-Methyl-1 -talloylamidoethyl-2-tallyl-imidazolinium-methosulfat.

Die Spiro-Verbindungen haben den besonderen Vorteil, daß sie auch bei Gegenwart von Aktivchlorspenden, wie Hypochlorit, wirksam sind und ohne wesentliche Einbuße der Effekte in Waschbädem mit nichtionogenen Waschmitteln, z. B. Alkylphenolpolyglykolethern, verwendet werden können.

Die Spiro-Verbindungen werden in Mengen von 0,00001 - 1% oder mehr, bezogen auf das Gewicht des flüssigen oder pulverförmigen, fertigen Waschmittels, zugesetzt. Waschflotten, die die angegebenen Mengen der beanspruchten optischen Aufheller enthalten, verleihen beim Waschen von Textilien aus Cellulosefasern, Polyamidfasern, hochveredelten Cellulosefasern, Polyesterfasern, Wolle etc. einen brillanten Aspekt am Tageslicht.

### Die Waschbehandlung wird beispielsweise wie folgt durchgeführt:

Die angegebenen Textilien werden während 1 bis 30 Minuten bei 20 bis 100 °C in einem Waschbad behandelt, das 1 bis 10 g/kg eines aufgebauten, zusammengesetzten Waschmittels und 0,0005 bis 1 %, bezogen auf das Waschmittelgewicht, der beanspruchten Aufhellmittel enthält. Das Flottenverhältnis kann 1 : 3 bis 1 : 50 betragen. Nach dem Waschen wird wie üblich gespült und getrocknet. Das Waschbad kann als Bleichzusatz 0,2 g/l Aktivchlor (z. B. Hypochlorit) oder 0,1 bis 2 g/l Natriumperborat enthalten.

Die Spiro-Verbindungen können auch im Nachspülbad, wie es zur bloßen Verleihung von Weichgriff, antistatischen Eigenschaften, Antisoileffekten, Duftnoten usw. üblich ist, eingesetzt werden. Insbesondere eignen sie sich für den Einsatz in Wäschenachbehandlungsmitteln, welche kationische Weichmacher enthalten.

### Beispiele

### A. Ausgangsverbindungen

a) Synthese von 9,9'-Spirobifluoren
   6,3 g Magnesiumspäne und 50 mg Anthracen wurden in 120 ml trockenem Diethylether in einem 1 l Dreihalskoben mit Rückflußkühler unter Argon vorgelegt und das Magnesium 15 min mit Ultraschall aktiviert:
   62 g 2-Brombiphenyl wurden in 60 ml trockenem Diethylether gelöst. Etwa 10 ml dieser Lösung wurden dem vorgelegten Magnesium zugegeben, um die Grignard-Reaktion zu starten.
   Nach dem Anspringen der Reaktion wurde unter weiterer Ultrabeschallung die 2-Brombiphenyl-Lösung so zugetropft, daß die Lösung gelinde am Rückfluß siedete. Nach beendeter Zugabe wurde die Reaktionsmischung eine weitere Stunde am Rückfluß unter Ultraschall gekocht.
   48,8 g 9-Fluorenon wurden in 400 ml trockenem Diethylether gelöst und unter weiterer Ultrabeschallung der Grignard-Lösung zugetropft. Nach beendeter Zugabe wurde weitere 2 h gekocht. Der nach Abkühlung der Reaktionsmischung ausgefallene gelbe Magnesiumkomplex des 9-(2-Biphenyl)-9-fluorenols wurde abgesaugt und mit wenig Ether gewaschen. Der Magnesiumkomplex wurde in 800 ml Eiswasser hydrolysiert, welches 40 g Ammoniumchlorid enthielt. Nach 60 min Rühren wurde das gebildete 9-(2-Biphenyl)-9-fluorenol abgesaugt, mit Wasser gewaschen und trocken gesaugt.
   Das getrocknete 9-(2-Biphenyl)-9-fluorenol wurde dann in 500 ml Eisessig in der Hitze gelöst. Zu dieser Lösung wurden 0,5 ml konz. Salzsäure gegeben. Man ließ die Lösung einige Minuten kochen und fällte das gebildete 9,9'-Spirobifluoren aus der heißen Lösung mit Wasser (Wasserzugabe bis Trübung einsetzte). Nach Abkühlung wurde das Produkt abgesaugt und mit Wasser gewaschen. Das getrocknete Produkt wurde zur weiteren Reinigung aus Ethanol umkristallisiert. Man erhielt 66 g (80 %, bez. auf 2-Brombiphenyl) 9,9'-Spirobifluoren als farblose Kristalle, Smp. 198°C.
b) 2,2'-Dibrom-9,9'-spirobifluoren
   (F. K. Sutcliffe, H. M. Shahidi, D. Patterson, J. Soc. Dyers Colour 94 (1978) 306)
   3,26 g (10,3 mmol) 9,9'-Spirobifluoren wurden in 30 ml Methylenchlorid gelöst und mit 5 mg FeCl₃ (wasserfrei) als Katalysator versetzt. Der Reaktionskolben wurde vor Lichtzutritt geschützt. 1,12 ml (21,8 mmol) Brom in 5 ml Methylenchlorid wurden innerhalb von 30 min unter Rühren zugetropft. Nach 24 h wurde die resultierende braune Lösung mit gesättigter wäßriger NaHCO₃-Lösung und Wasser gewaschen, um überschüssiges Brom zu entfernen. Die organische Phase wurde nach dem Trocknen über Na₂SO₄ am Rotationsverdampfer eingeengt. Der weiße Rückstand wurde aus Methanol umkristallisiert, man erhält 3,45 g (70 %) der Dibromverbindung als farblose Kristalle, Smp. 240°C.
c) 2,2',7,7'-Tetrabrom-9,9'-spirobifluoren
   Zu einer Lösung von 3,16 g (10,0 mmol) 9,9'-Spirobifluoren in 30 ml Methylenchlorid wurden 80 mg (0,5 mmol) wasserfreies FeCl₃ gegeben und 2,1 ml (41 mmol) Brom in 5 ml Methylenchlorid über 10 min zugetropft. Die Lösung wurde 6 h rückflußgekocht. Beim Abkühlen fiel das Produkt aus. Der Niederschlag wurde abgesaugt und mit wenig kaltem Methylenchlorid gewaschen. Nach dem Trocknen erhielt man 6,0 g (95 %) der Tetrabromverbindung als weißen Feststoff.
d) 2-Bromo-9,9'-spirobifluoren und 2,2',7-Tribrom-9,9'-spirobifluoren wurden bei veränderter Stöchiometrie auf analoge Weise hergestellt.
e) 9,9'-Spirobifluoren-2,2'-dicarbonsäure
   aus 2,2'-Dibrom-9,9'-spirobifluoren über 2,2'-Dicyano-9,9'-spirobifluoren
   1,19 g 2,2'-Dibromo-9,9'-spirobifluoren und 0,54 g CuCN wurden in 5 ml DMF 6 h zum Rückfluß erhitzt. Die erhaltene braune Mischung wurde in eine Mischung aus 3 g FeCl₃ (hydrat.) und 1,5 ml konz. Salzsäure in 20 ml Wasser gegossen. Die Mischung wurde 30 min bei 60 bis 70°C gehalten, um den Cu-Komplex zu zerstören. Die heiße wäßrige Lösung wurde zweimal mit Toluol extrahiert. Die organischen Phasen wurden dann mit verdünnter Salzsäure, Wasser und 10 %iger wäßriger NaOH gewaschen. Die organische Phase wurde filtriert und eingeengt. Der erhaltene gelbe Rückstand wurde aus Methanol umkristallisiert. Man erhielt 0,72 g (80 %) 2,2'-Dicyano-9,9'spirobifluoren als schwach gelbliche Kristalle (Schmelzbereich 215 bis 245°C).
   3 g 2,2'-Dicyano-9,9'-spirobifluoren wurden mit 25 ml 30 %iger wäßriger NaOH und 30 ml Ethanol für 6 h unter Rückfluß erhitzt. Das Dinatriumsalz der Spirobifluorendicarbonsäure fiel als gelber Niederschlag aus, der abfiltriert und in 25 %iger wäßriger HCl erhitzt wurde, um die freie Säure zu gewinnen. Die Spirobifluorendicarbonsäure wurde aus Eisessig umkristallisiert. Man erhielt 2,2 g (66,6 %) weiße Kristalle (Smp. 376°C, IR-Bande 1685 cm⁻¹ C=O).
   9,9'-Spirobifluoren-2,2',7,7'-tetracarbonsäure wurde aus 2,2',7,7'-Tetrabrom-9,9'-spirobifluoren in analoger Weise hergestellt.
f) 9,9'-Spirobifluoren-2,2'-dicarbonsäure aus 9,9'-Spirofluoren über 2,2'-Diacetyl-9,9'-spirobifluoren (G. Haas, V. Prelog, Helv. Chim. Acta 52 (1969) 1202; V. Prelog, D. Bedekovic, Helv. Chim. Acta 62 (1979) 2285)
   Eine Lösung von 3,17 g 9,9'-Spirobifluoren in 30 ml abs. Schwefelkohlenstoff wurde nach Zugabe von 9,0 g feingepulvertem, wasserfreiem AlCl₃ während 10 min tropfenweise unter Rühren mit 1,58 g Acetylchlorid in 5 ml abs. Schwefelkohlenstoff versetzt und 1 Stunde unter Rückfluß gekocht. Die unter vermindertem Druck zur Trockene eingedampfte Mischung wurde bei 0°C mit 100 g Eis und 50 ml 2n Salzsäure versetzt. Nach üblicher Aufarbeitung wurde das Rohprodukt chromatographisch mit Benzol/Essigester (10: 1) an Kieselgel getrennt. Man erhielt 3,62 g (89 %) 2,2'-Diacetyl-9,9'-spirobifluoren (umkristallisiert aus Chloroform/Essigester, Smp. 255 bis 257°C) und 204 mg 2-Acetyl-9,9'-spirobifluoren (umkrist. aus Chloroform/Benzol, Smp. 225°C). [Daneben konnten bei der Chromatographie auch 2,2',7-Triacetyl-9,9'-spirobifluoren (Smp. 258 bis 260°C) und 2,2',7,7'-Tetraacetyl-9,9'-spirobifluoren (Smp. > 300°C) isoliert werden, umkristallisiert aus Essigester/Hexan].
   2,2',7-Triacetyl- und 2,2',7,7'-Tetraacetyl-9,9'-spirobifluoren 2,2',7,7'-Tetraacetyl-9,9'-spirobifluoren wurden bei veränderter Stöchiometrie als Hauptprodukt erhalten.
   Zu einer Lösung von 6,0 g Natriumhydroxid in 30 ml Wasser wurden bei 0°C unter Rühren zuerst 7,2 g Brom und dann eine Lösung von 3,0 g 2,2'-Diacetyl-9,9'-spirobifluoren in wenig Dioxan zugetropft. Nach weiterem 1 stündigen Rühren bei Raumtemperatur wurde die klare gelbe Lösung mit 1 g Natriumhydrogensulfit, gelöst in 20 ml Wasser, versetzt. Nach Ansäuern mit konz. Salzsäure wurde das ausgefallene farblose Produkt abfiltriert und mit wenig Wasser gewaschen. Umkristallisation aus Ethanol lieferte 9,9'-Spirobifluoren-2,2'-dicarbonsäure als wasserklare Prismen (Smp 352°C).
   9,9'-Spirobifluoren-2-carbonsäure, 9,9'-Spirobifluoren-2,2',7-tricarbonsäure und 9,9'-Spirobifluoren-2,2',7,7'-tetracarbonsäure wurden in analoger Weise hergestellt.
g) 2,2'-Bis(brommethyl)-9,9'-spirobifluoren
   aus 2,2'-Dicarboxy-9,9'-spirobifluoren über 9,9'-Spirobifluoren-2,2'-dimethanol (V. Prelog, D. Bedekovicc, Helv. Chim. Acta 62 (1979) 2285)
   Bei Raumtemperatur wurden 10 g einer 70 gew.-%igen Lösung von Natriumdihydro-bis(2-methoxyethoxy)-aluminat (Fluka) in Benzol langsam zu einer Suspension von 2,0 g 2,2'-Dicarboxy-9,9'-spirobifluoren (freie Carbonsäure) in 20 ml Benzol zugetropft. Nach 2 h Kochen unter Rückfluß, wobei sich die Carbonsäure auflöste, wurde das überschüssige Reduktionsmittel bei 10°C mit Wasser zersetzt, das Gemisch mit konz. Salzsäure angesäuert und mit Chloroform ausgeschüttelt.
   Die mit Wasser gewaschene und über Magnesiumsulfat getrocknete organische Phase wurde eingedampft und der Rückstand aus Benzol umkristallisiert. Man erhielt 1,57 g 9,9'-Spirobifluoren-2,2'-dimethanol (Smp. 254 bis 255°C). Zu einer Lösung von 13,5 g 9,9'-Spirofluoren-2,2'-dimethanol in 400 ml Benzol wurden 91,5 g einer 33 %igen wäßrigen Lösung von Bromwasserstoff in Eisessig zugetropft und das Gemisch 7 h unter Rückfluß gekocht. Danach wurde mit 200 ml Wasser versetzt und die mit Wasser gewaschene und über Magnesiumsulfat getrocknete organische Phase eingedampft. Die Chromatographie an Kieselgel mit Benzol lieferte 11,7 g 2,2'-Bis(brommethyl)-9,9'-spirobifluoren als farblose Plättchen (Smp. 175 bis 177°C).
h) Eine Lösung von 380 mg 9,9'-Spirobifluoren-2,2'-dimethanol in 15 ml Toluol wurde mit 5 g Chrom(VI)oxid auf Graphit (Seloxcette, Alpha Inorganics) versetzt und 48 h unter Stickstoff am Rückfluß gekocht. Dann wurde durch eine Glasfilternutsche abgenutscht und das Filtrat eingedampft. Chromatographie an Kieselgel mit Chloroform und Kristallisation aus Methylenchlorid/Ether lieferte 152 mg 9,9'-Spirobifluoren-2,2'-dicarbaldehyd (Smp. > 300°C) und 204 mg 2'-Hydroxymethyl-9,9'-spirobifluoren-2-carbaldehyd (Smp. 262 bis 263°C).
i) 2,2'-Diamino-9,9'-spirobifluoren
   Eine Mischung aus 150 ml konz. wäßriger HNO₃ und 150 ml Eisessig wurden zu einer kochenden Lösung von 15,1 g 9,9'-Spirobifluoren in 500 ml Eisessig über einen Zeitraum von 30 min zugetropft anschließend wurde die Lösung 75 min weiter refluxiert. Nach Abkühlung und Stehenlassen der Lösung für 1 h wurde das gleiche Volumen Wasser zugesetzt und damit das Produkt ausgefällt. Nach dem Absaugen erhielt man 18,5 g gelbe Kristalle (Smp. 220 bis 224°C) von 2,2'-Dinitro-9,9'-Spirobifluoren. Umkristallisation aus 250 ml Eisessig ergab 12,7 g hellgelbe Kristallnadeln (Smp. 245 bis 249°C, analytisch rein 249 bis 250°C).
   Eine Mischung aus 4,0 ml Dinitro-spriobifluoren und 4,0 g Eisenpulver wurden in 100 ml Ethanol unter Rückfluß erhitzt, während 15 ml konz. HCl über einen Zeitraum von 30 min zugetropft wurden. Nach weiteren 30 min Rückflußkochen wurde überschüssiges Eisen abfiltriert. Das grüne Filtrat wurde in eine Lösung aus 400 ml Wasser, 15 ml konz. NH₄OH und 20 g Na,K-Tartrat gegeben. Das weiße Diamin wurde von der dunkelgrünen Lösung des Eisenkomplexes abfiltriert. Das Diamin wurde zur Reinigung in verdünnter HCl gelöst und bei Raumtemperatur mit Aktivkohle (Darco) gerührt und abfiltriert. Die filtrierte Lösung wurde unter Rühren (KPG-Rührer) tropfenweise mit NH₄OH neutralisiert und das ausgefallene Produkt abgesaugt. Man erhielt 3,5 g weißes 2;2'-Diamino-9,9'-spirobifluoren, das aus Ethanol umkristallisiert wurde (Smp. 243°C).
j) Synthese von 2,2',7,7'-Tetrabromo-9,9'-spirobifluoren durch Bromierung von festem 9,9'-Spirobifluoren mit Bromdampf.
   In eine flache Porzellan-Abdampfschale (ø2 ca. 15 cm) wurden 3.16 g (10 mmol) fein gepulvertes 9,9'-Spirobifluoren gegeben. Diese Schale wurde in einen Exsikkator (∅ ca. 30 cm), auf den gelochten Zwischenboden gestellt. Auf dem Boden des Exsikkators befanden sich ein einer Kristallisierschale 15.6 g (4.8 ml ,96 mmol) Brom. Der Exsikkator wurde verschlossen, der Belüftungshahn jedoch geöffnet, damit das gebildete HBr entweichen konnte. Der Exsikkator wurde über Nacht in den Abzug gestellt. Am nächsten Tag wurde die Porzellanschale mit dem durch Brom orange gefäbten Produkt aus dem Exsikkator genommen, und im Abzug noch mindestens 4 h stehen gelassen, damit überschüssiges Brom und HBr entweichen konnte.
   Das Produkt wurde in 150 ml Dichlormethan gelöst und mit je 50 ml Natriumsulfitlösung (gesättigt), Natriumhydrogenkarbonatlösung (gesättigt) und Wasser farblos gewaschen. Die Dichlormethanlösung wurde über Natriumsulfat getrocknet und am Rotationsverdampfer eingeengt. Zur Reinigung wurde aus Dichormethan/Pentan 4:1 umkristallisiert. Ausbeute 5.7 g (92%) farblose Kristalle.
   ¹H-NMR (CDCl₃, ppm): 6.83 (d, J = 1,83 Hz, 4 H, H-1,1',8,8'); 7.54 (dd, J = 7.93, 1.83 Hz, 4 H, H-3,3',6,6'); 7.68 (d, J = 7.93 Hz, 4 H, H-4,4',5,5').
k) Synthese von 2,2',4,4',7,7'-Hexabromo-9,9'-spirobifluoren
   Zu einer Lösung von 3.16 g (10 mmol) 9,9'-Spirobifluoren in 20 ml Methylenchlorid wurden 200 mg wasserfreies FeCl₃ gegeben und mit Ultraschall behandelt. Der Reaktionskolben wurde mit Al-Folie vor Lichtzutritt geschützt. Anschließend wurden in der Siedehitze 9.85 g (3.15 ml, 62 mmol) Brom in 5 ml Methylenchlorid innerhalb von 15 min zugetropft. Die Lösung wurde weitere 20 h am Rückfluß gekocht und mit Ultraschall behandelt. Nach Abkühlung wird Petrolether versetzt und abgesaugt. Zur weiteren Reinigung wurde aus THF / Methanol umkristallisiert und 5 h bei 80°C getrocknet. Ausbeute 6.15 g (77 %) farblose Kristalle.
   ¹H-NMR (CDCl₃, ppm): 6.76 (d, J = 1,53 Hz, 2 H, H-1,1'); 6.84 (d, J = 1.83 Hz, 2H, H-8,8'); 7.60 (dd, J = 8.54, 1.83 Hz, 2 H, H-6,6'); 7.75 (d, J = 1.53 Hz, 2 H, H-3,3'); 8.49 (d, J = 8.54 Hz, 2 H, H-5,5').
l) Synthese von 2,7-Dibromo-9,9'-spirobifluoren
   Ein Grignardreagenz bereitet aus 0.72 g (30 mmol) Magnesiumspänen und 5.1 ml (30 mmol) 2-Brombiphenyl in 15 ml Diethylether wurde im Verlauf von 2 h unter Rühren (im Ultraschallbad) zu einer siedenden Suspension von 10.0 g (29.6 mmol) 2,7-Dibrom-9-fluorenon in 100 ml trockenem Diethylether getropft. Nach beendeter Zugabe wurde 3 Stunden weiter gekocht. Nach Abkühlung über Nacht wurde der ausgefallene Niederschlag abgesaugt und mit kaltem Ether gewaschen. Der abgesaugte Magnesiumkomplex wurde in einer Lösung von 15 g Ammoniumchlorid in 250 ml Eiswasser hydrolysiert. Nach 1 h wurde das gebildete 9-(2-Biphenylyl)-2,7-dibromo-9-fluorenol abgesaugt, mit Wasser gewaschen und trockengesaugt. Das getrocknete Fluorenol wurde für die Ringschlußreaktion in 100 ml Eisessig, nach Zugabe von 3 Tropfen HCl conc. 6 Stunden gekocht. Man ließ über Nacht kristallisieren, saugte das gebildete Produkt ab und wusch mit Eisessig und Wasser.
   Ausbeute: 11 g ( 77 %) 2,7-Dibromo-9,9'-spirobifluoren. Zur weiteren Reinigung wurde aus THF umkristallisiert.
   ¹H-NMR (CDCl₃, ppm): 6.73 (d, J= 7.63 Hz, 2 H, H-1',8'); 6.84 (d, J = 1.83 Hz, 2 H, H-1,8); 7.15 (td, J = 7.63, 1.22 Hz., 2 H, H-2',7'); 7.41 (td, J = 7.63, 1.22 Hz, 2 H, H-3',6'); 7.48 (dd, J = 8.24, 1.83 Hz, 2 H, H-3,6); 7.67 (d, J = 8.24; 2 H; H-4,5); 7.85 (d, J = 7.63, 2 H, H-4',5').
m) Synthese von 2,7-Dicarbethoxy-9,9'-spirobifluoren
   Ein Grignardreagenz bereitet aus 0.97 g (40 mmol) Magnesiumspänen und 9.32 g (6.8 ml, 40 mmol) 2-Brombiphenyl in 50 ml trockenem Diethylether wurde im Verlauf von 2 h zu einer siedenden Lösung von 13 g (40 mmol) 2,7-Dicarbethoxy-9-fluorenon in 100 ml trockenem Diethylether getropft. Nach beendeter Zugabe wurde 3 Stunden weiter gekocht. Nach Abkühlung über Nacht wurde der ausgefallene Niederschlag abgesaugt und mit kaltem Ether gewaschen. Der abgesaugte Magnesiumkomplex wurde in einer Lösung von 15 g Ammoniumchlorid in 250 ml Eiswasser hydrolysiert. Nach 1 h wurde das gebildete 9-(2-Biphenylyl)-2,7-dicarbethoxy-9-fluorenol abgesaugt, mit Wasser gewaschen und trockengesaugt. Das getrocknete Fluorenol wurde für die Ringschlußreaktion in 100 ml Eisessig, nach Zugabe von 3 Tropfen HCl conc. 6 Stunden gekocht. Man ließ über Nacht kristallisieren, saugte das gebildete Produkt ab und wusch mit Eisessig und Wasser.
   Ausbeute: 15.1 g (82 %) 2,7-Dicarbethoxy-9,9'-spirobifluoren. Zur weiteren Reinigung wurde aus Ethanol umkristallisiert.
   ¹H-NMR (CDCl₃, ppm): 1.30 (t, J = 7.12 Hz, 6 H, Ester-CH₃); 4.27 (q, J = 7.12 Hz, 4 H, Ester-CH₂); 6.68 (d, J = 7.63 Hz, 2 H, H-1', 8'); 7.11 (td, J = 7.48, 1.22 Hz, 2H, H-2', 7'); 7.40 ( td, J = 7.48, 1.22 Hz, 4 H, H-1, 8, 3', 6'); 7.89 (dt, J = 7.63, 0.92 Hz, 2 H, H-4', 5'); 7.94 (dd, J = 7.93, 0.6 Hz, 2 H, H-4, 5); 8.12 (dd, J = 7.93, 1.53 Hz, 2 H, H-3, 6).
n) Synthese von 2,7-Dibromo,2',7'-dijodo-9,9'-spirobifluoren
   In einem 250 ml Dreihalskolben mit Rückflußkühler und Tropftrichter wurde bei 80°C eine Suspension von 2.37 g 2,7-Dibrom-9,9'-spirobifluoren in 50 ml Eisessig mit 5 ml Wasser versetzt und nach Zugabe von 2 ml konz. Schwefelsäure, 1.27 g lod, 0.53 g lodsäure sowie 5 ml Tetrachlorkohlenstoff bis zum Verschwinden der lodfarbe gerührt. Anschließend wurde abgesaugt und gut mit Wasser gewaschen. Nach dem Trocknen wurde der Niederschlag in 150 ml Dichlormethan gelöst, und nacheinander mit Na₂SO₃-Lösung, NaHCO₃-Lösung und mit Wasser gewaschen. Die Dichlormethanphase wurde über Na₂SO₄ getrocknet und anschließend eingeengt. Man erhielt farblose Kristalle von 2,7-Dibromo,2',7'-dijodo-9,9'spirobifluoren in quantitativer Ausbeute. Zur weiteren Reinigung wurde aus Dichlormethan/Pentan umkristallisiert.
   ¹H-NMR (CHCl₃, ppm):
   6,80 (d, J = 1.83 Hz, 2 H), 6.99 (d, J = 1.53 Hz, 2 H), 7.51 (dd, J = 8.24, 1.83 Hz, 2 H), 7.54 (d, J = 7.93 Hz, 2 H), 7.65 (d, J = 8.24 Hz, 2 H), 7.72 (dd, J = 8.24, 1.53 Hz, 2 H).

### B. Synthesebeispiele

### Beispiel 1

### 2,2'-Bis(benzofuran-2-yl)-9,9'-spirobifluoren (analog zu W.Sahm, E.Schinzel, P.Jürges, Liebigs Ann.Chem. (1974) 523.)

2,7 g (22 mmol) Salicylaldehyd und 5,0 g (10 mmol) 2,2'-Bis(brommethyl)-9,9'spirobifluoren wurden bei Raumtemperatur in 15 ml DMF gelöst und mit 0,9 g (22,5 mmol) pulverisiertem NaOH sowie einer Spatelspitze Kl versetzt. Man erhitzte zum Sieden und rührte 1 h bei Siedetemperatur. Nach Abkühlung versetzte man die Reaktionslösung mit einem Gemisch aus 0,5 ml konz. Salzsäure, 7 ml Wasser und 7 ml Methanol. Man rührte noch 1 h bei Raumtemperatur, saugte die kristallinen Reaktionsprodukte ab, wusch zunächst mit kaltem Methanol, dann mit Wasser und trocknete im Vakuum bei 60°C. Man erhielt 4,6 g (79 %) des Bisbenzylphenylethers. 5,85 g (10 mmol) des Bisbenzylphenylethers wurden in 10 ml Toluol mit 2,1 g (22,5 mmol) frisch destilliertem Anilin versetzt. Man gab eine Spatelspitze p-Toluolsulfonsäure zu und erhitzte am Wasserabscheider so lange zum Sieden, bis sich kein Wasser mehr abtrennte (ca. 3 bis 5 h). Beim Abkühlen des Reaktionansatzes fiel das korrespondierende bis-Benzylidenphenylamin kristallin aus. Es wurde abgesaugt, mit Methanol gewaschen und im Vakuum bei 60°C getrocknet. Zur weiteren Reinigung wurde aus DMF umkristallisiert.
7,35 g (10 mmol) des bis-Benzylidenphenylamins und 0,62 g (11 mmol) KOH wurden unter Stickstoff in 30 ml DMF eingetragen. Anschließend erhitzte man unter Rühren 4 h auf 100°C. Nach Abkühlung auf Raumtemperatur wurde der Niederschlag abgesaugt und mit wenig DMF und Wasser gewaschen. Nach Trocknen bei 60°C im Vakuumtrockenschrank wurde das 2,2'-Bis(benzofuran-2-yl)-9,9'-spirobifluoren durch Umkristallisation aus Benzoesäuremethylester gereinigt.

### Beispiel 2

2,2',7,7'-Tetra(benzofuran-2-yl)-9,9'-spirobifluoren
wurde bei entsprechend veränderter Stöchiometrie analog zu Beispiel 1 hergestellt.

### Beispiel 3

### 2,2',7,7'-Tetraphenyl-9,9'-spirobifluoren

5 g (7,9 mmol) 2,2',7,7'-Tetrabrom-9,9'-spirobifluoren, 3,86 g (31,6 mmol) Phenylboronsäure, 331,5 mg (1,264 mmol) Triphenylphosphan und 70,9 mg (0,316 mmol) Palladiumacetat wurden in einer Mischung aus 65 ml Toluol und 40 ml wäßriger Natriumcarbonatlösung (2 M) aufgeschlämmt. Unter starkem Rühren wurde die Mischung 24 h am Rückfluß gekocht. Nach Abkühlung auf Raumtemperatur wurde abgesaugt, mit Wasser gewaschen und bei 50C im Vakuum getrocknet. Man erhielt 2,58 g Produkt. Das Filtrat wurde mit 50 ml Toluol extrahiert und die getrocknete organische Phase zur Trockene eingeengt. Man erhielt weitere 1,67 g Produkt.
Gesamtausbeute 4,25 g (86 %)

### Beispiel 4

### Synthese von 2,2',7,7'-Tetrakis(biphenylyl-4-yl)-9,9-spirobifluoren

In einem 250 ml Zweihalskolben mit Rückflußkühler und KPG-Rührer wurden 5,5 g Tetrabromspirobifluoren, 7.2 g Biphenylboronsäure und 400 mg Tetrakis(triphenylphosphin)palladium, in einer Mischung aus 100 ml Toluol und 50 ml Kaliumcarbonatlösung aufgeschlämmt. Unter Rühren mit einem KPG-Rührer und Schutzgasüberlagerung wurde die Mischung 8 h am Rückfluß gekocht. Nach Abkühlung wurde das Produkt abgesaugt, der Niederschlag mit Wasser gewaschen und getrocknet. Im Filtrat wurde die Toluol-Phase abgetrennt und die wäßrige Phase einmal mit Chloroform ausgeschüttelt. Die vereinigten organischen Phasen wurden über Natriumsulfat getrocknet und am Rotationsverdampfer eingeengt; man erhielt so eine zweite Fraktion des Produktes. Die beiden Produktfraktionen wurden vereinigt (8g) und in Chlorofrom gelöst. Die Chloroformlösung wurde mit Aktivkohle aufgekocht und über eine kurze Säule mit Kieselgel filtriert. Nach Einengen am Rotationsverdampfer und Umkristallisation aus Chloroform / Pentan erhielt man farblose, im UV-Licht blau fluoreszierende Kristalle. Schmelzpunkt 408°C (DSC).
¹H-NMR (CDCl₃, ppm): 7.14 (d, J = 1,53 Hz, 4 H ); 7.75 (dd, J = 7.93, 1.53 Hz, 4 H); 8.01 (d, J = 7.93 Hz, 4 H); 7.34 (dd, J = 7.32, 1.37 Hz, 4 H); 7.42 (t, J = 7.32 Hz, 8 H); 7.58 (24 H).

### Beispiel 5

### Synthese von 2,2',4,4',7,7'-Hexakis(biphenyl-4-yl)-9,9-spirobifluoren

In einem 250 ml Zweihalskolben mit Rückflußkühler, KPG-Rührer wurden 1,6 g Hexabromspirobifluoren und 3 g Biphenylboronsäure in einer Mischung aus 50 ml Toluol und 50 ml 1 M Kaliumcarbonatlösung aufgeschlämmt. Die Mischung wurde unter Stickstoff am Rückfluß gekocht und 115 mg Tetrakis(triphenylphosphan)-palladium in 5 ml Toluol wurden zugegeben. Die Mischung wurde unter Rühren 7 h am Rückfluß gekocht. Nach Beendigung der Reaktion wurde die abgekühlte Lösung abfiltriert und das Filtrat 2 x mit Wasser ausgeschüttelt (zur besseren Phasentrennung wurde Chloroform zugesetzt). Die organische Phase wurde über Natriumsulfat getrocknet, über eine kurze Säule mit Kieselgel filtriert und anschließend eingeengt. Zur weiteren Reinigung wurde aus Dichlormethan / Pentan umkristallisiert. Man erhielt 2 g (80 %) farblose, im UV-Licht blau fluoreszierende Kristalle.
¹³C-NMR [360 MHz.; ATP, breitbandentkoppelt] (CDCl₃, ppm):
65.94 (1C, Spiro-C); 126.95 (6C, CH), 126.97 (6C, CH), 127.17 (6C, CH), 127.35 (6C, CH), 127.36 (6C, CH), 127.39 (6C, CH), 127.52 (6C, CH), 128.73 (6C, CH), 128.75 (6C, CH), 128.94 (6C, CH), 129.90 (4 C, CH), 137.77 (2 C), 137.86 (2 C), 139.43 (2 C), 139.69 (2 C), 139.89 (2 C), 140.09 (2 C), 140.17 (2 C), 140.22 (2 C), 140.30 (2 C), 140.63 (2 C), 140.64 (2 C), 140.68 (2 C), 140.72 (2 C), 140.74 (2 C), 150.45 (2C), 150.92 (2C).

### Beispiel 6

### Synthese von 2,2'-Bis[(5(p-t-butylphenyl)-1,3,4-oxadiazol-2yl]-9,9'-spirobifluoren aus 9,9'-Spirobifluoren-2,2'-dicarbonsäurechlorid und 5(4-t-Butylphenyl)tetrazol

a) Synthese von 5(4-t-Butylphenyl)tetrazol
   In einem 250 ml Rundkolben mit Rückflußkühler wurden 4,9 g p-t-Butylbenzonitril, 3,82 g Lithiumchlorid und 5,85 g Natriumazid und 8,2 g Triethylammoniumbromid in 100 ml DMF für 8 h auf 120°C erhitzt. Nach Abkühlung auf Raumtemperatur wurden 100 ml Wasser zugesetzt und im Eisbad mit verd. Salzsäure versetzt, bis kein weiterer Niederschlag mehr ausfiel. Es wurde abgesaugt, der Niederschlag mit Wasser gewaschen und getrocknet. Umkristallisation aus Ethanol / Wasser lieferte 4,4 g farblose Kristalle.
b) 9,9'-Spirobifluoren-2,2'-dicarbonsäurechlorid
   In einem 100 ml Kolben mit Rückflußkühler und Trockenrohr wurden 2 g (5 mmol) 9,9'-Spirobifluoren-2,2'-dicarbonsäure mit 20 ml (frisch destilliertem) Thionylchlorid und 3 Tropfen DMF 4 h am Rückfluß gekocht. Nach Abkühlung wurde der Rückflußkühler gegen eine Destillationsbrücke ausgetauscht und überschüssiges Thionylchlorid im Vakuum abdestilliert; dem Rückstand wurden 40 ml Petrolether (30°-60°C) zugesetzt und abdestilliert, zurück blieb das kristalline Säurechlorid.
c) 2,2'-Bis[(5(*p*-*t*-butylphenyl)-1,3,4-oxadiazol-2yl]-9,9'-spirobifluoren
   Dem Säurechlorid wurden 2,0 g (11 mmol) 5(4-*t*-Butylphenyl)tetrazol gelöst in 20 ml wasserfreiem Pyridin zugesetzt und unter Schutzgas 2 h zum Rückfluß erhitzt. Nach Abkühlung wurde die Mischung in 200 ml Wasser gegeben und 2 h stehen gelassen. Das ausgefallene Oxadiazolderivat wurde abgesaugt, mit Wasser gewaschen und im Vakuum getrocknet. Anschließend wurde über Kieselgel mit Chloroform/Essigester (99:1) chromatographiert und aus Chloroform / Pentan umkristallisiert. Man erhielt 2,4 g farblose Kristalle.
   ¹H-NMR (CDCl₃), ppm):
   1.31 (s, 18 H, t-Butyl), 6.77 (d, J = 7.32 Hz, 2 H), 7.18 (td, J = 7.48, 1.22 Hz, 2 H), 7.44 (td, J = 7.40, 1.22 Hz, 2 H), 7.46 (d, J = 8.54 Hz, 4 H), 7.50 (d, J = 1.22 Hz, 2 H), 7.94 (d, J = 8.54 Hz, 4 H), 8.02 (d, J = 7.93 Hz, 6 H), 8.20 (dd, J = 7.93, 1.53 Hz, 2 H).

### C. Anwendungsbeispiele

### Beispiel 1

100 Teile Polyester-Granulat aus Polyterephthalsäureethylenglykolester wurden innig mit 0,05 Teilen der Verbindung der Formel (A) vermischt und bei 285°C unter Rühren geschmolzen.
Nach dem Ausspinnen der Spinnmasse durch übliche Spinndüsen wurden stark aufgehellte Polyesterfasern erhalten. Verwendete man anstelle der Verbindung der Formel (A) eine solche der Formel (B) oder(C) so erhielt man ähnliche Ergebnisse.

### Beispiel 2

Man formulierte bei Raumtemperatur ein Polyestergewebe ("Dacron") mit einer wäßrigen Dispersion, die im Liter 2 g der Verbindungen der Formel (B) sowie 1 g eines Anlagerüngsproduktes aus etwa 8 mol Ethylenoxyd an 1 Mol p-tert.-Octylphenol enthielt und trocknete bei etwa 100°C. Das trockene Material wurde anschließend kurze Zeit einer Wärmebehandlung von 220°C unterworfen. Das derart behandelte Material hatte ein wesentlich weißeres Aussehen als das unbehandelte Material. Setzte man anstelle der Verbindung der Formel (B) eine solche der Formel (A) oder (C) ein, so erhielt man ähnliche Ergebnisse.

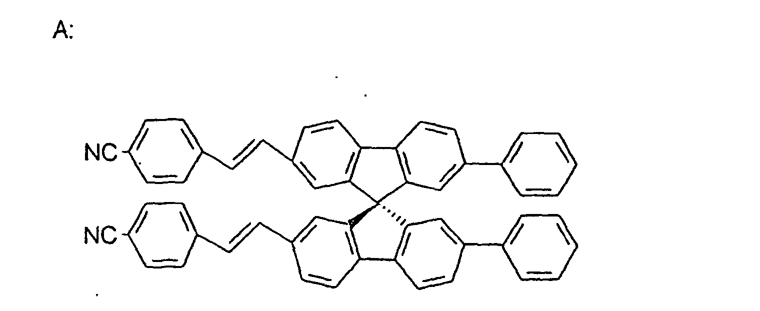

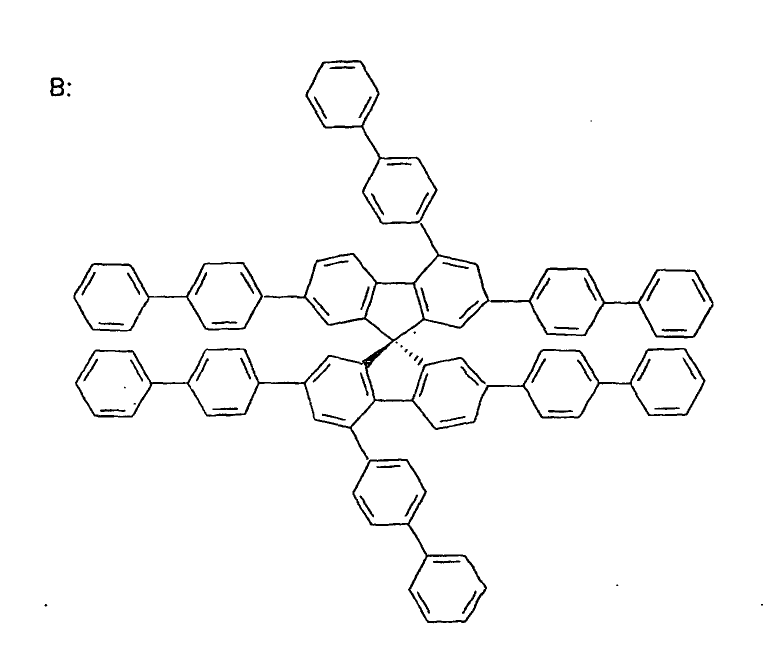

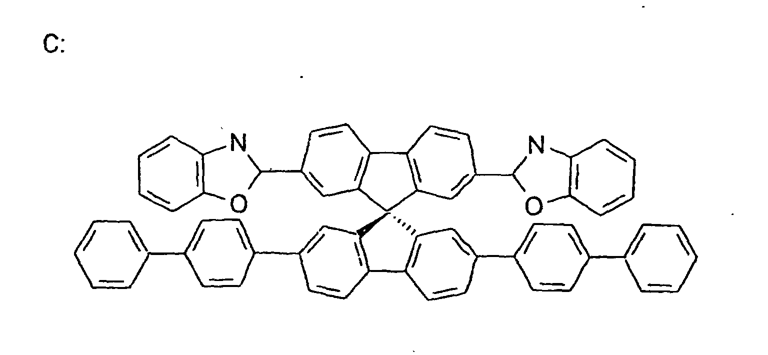

**Tabelle 1:**

| Spektrale Daten ausgewählter optischer Aufheller | | | |
|---|---|---|---|
| Verbindung | Absorption λₘₐₓ [nm] | Extinktion Ig ε | Emission λₘₐₓ [nm] |
| 1 | 334 | 4,85 | 359,378 |
| 2 | 342 | 5,11 | 385, 406 |
| 3 | 344 | 5,23 | 395,416 |
| 4 | 344 | | 405, 422 |
| 5 | 353 | | 391,412 |
| 6 | 363 | | 392,413 |
| 7 | 330 | | 355, 373, 393 |

**Tabelle 2:**

| Thermochemische Daten ausgewählter Spiro-Verbindungen | | |
|---|---|---|
| Verbindung | Schmelzpunkt [°C] | Thermischer Abbau (5 % Gewichtsverlust) |
| 1 | 296 | 425 |
| 2 | 408 | 550 |
| 3 | 438 | 585 |
| 8 | 316 | 465 |
| 6 | 365 | 565 |
| 7 | 337 | - |

- Vbdg. 1:: 2,2',7,7'-Tetraphenyl-9,9'-spirobifluoren (Bsp. 3)
- Vbdg. 2:: 2,2',7,7'-Tetrakis(biphenyl-4-yl)-9,9'-spirobifluoren (Bsp. 4)
- Vbdg. 3:: 2,2',7,7'-Tetrakis(terphenyl-4-yl)-9,9'-spirobifluoren
- Vbdg. 4:: 2,2',7,7'-Tetrakis(quaterphenyl-4-yl)-9,9'-spirobifluoren
- Vbdg. 5:: 2,2',7,7'-Tetrakis(4'-methoxybiphenyl)-4-yl)-9,9'-spirobifluoren
- Vbdg. 6:: 2,2',4,4',7,7'-Hexakis(biphenyl-4-yl)-9,9'-spirobifluoren
- Vbdg. 7:: 2,2'-Bis[(5(p-t-butylphenyl-1,3,4-oxadiazol-2-yl]-9,9'-spirobifluoren
- Vbdg. 8:: 2,2',4,4',7,7'-Hexaphenyl-9,9'-spirofluoren

## Patentansprüche

1. Verwendung eines Spirobifluorenderivat der Formel (III), 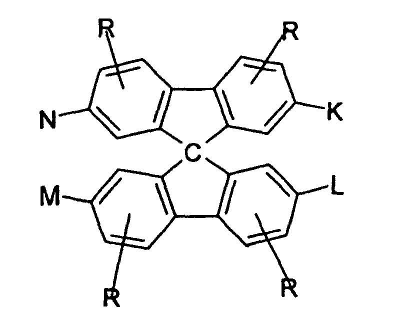 wobei K, L, M, N gleich oder verschieden sind und eine Gruppe der folgenden Formeln bedeuten: 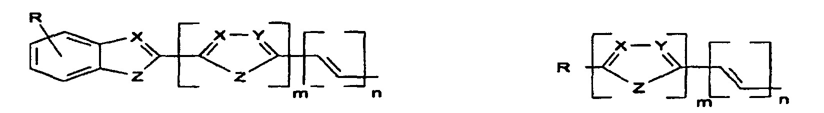 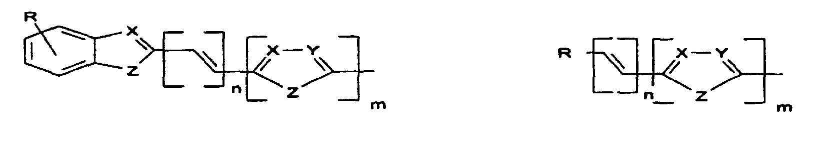 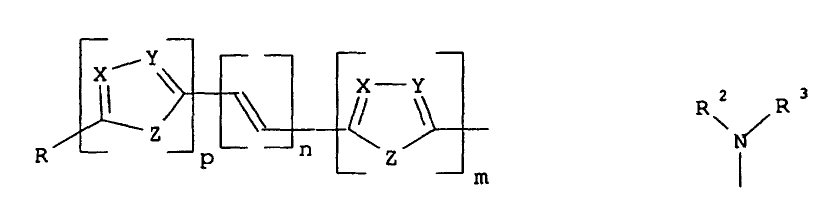
R gleich oder verschieden ist und für Wasserstoff, eine lineare oder verzweigte Alkyl, Alkoxy oder Carboalkoxygruppe mit 1 bis 22 C-Atomen, -CN, -NO₂, -NR²R³, -N⁺R²R³R⁴, NOR²R³, -Ar oder -O-Ar;
Ar ist Phenyl, Biphenyl, 1-Naphthyl, 2-Naphthyl, 2-Thienyl, 2-Furanyl,
m, n, p sind 0, 1, 2 oder 3, 4, 5;
X, Y sind gleich oder verschieden CR oder N;
Z ist -O-, -S-, -NR-, -CR¹R-, -CH=CH-, -CH=N-;
R¹ hat die gleiche Bedeutung wie R;
R², R³, R⁴ sind gleich oder verschieden und bedeuten H, eine lineare oder verzweigte
R², R³, R⁴ sind gleich oder verschieden und bedeuten H, eine lineare oder verzweigte Alkylgruppe mit 1 bis 22 C-Atomen, -Ar, 3-Methylphenyl,
als optischer Aufheller.

2. Verwendung gemäß Anspruch 1, **dadurch gekennzeichnet, daß** ein Spirobifluorenderivat der Formel (IIIa) bis (IIIg):
IIIa) K = L = M = N und ist eine Gruppe der Formel: 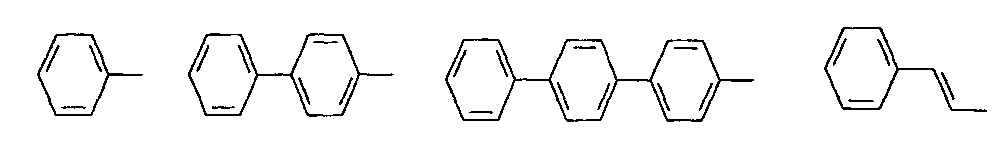 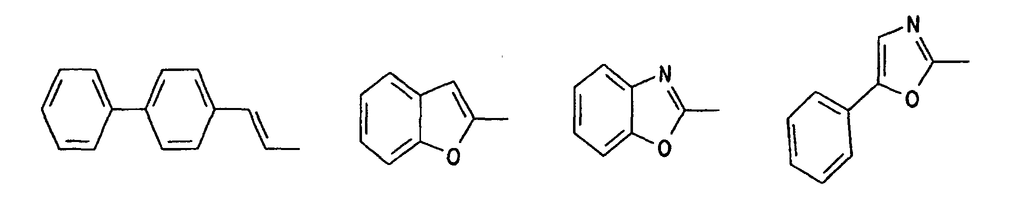 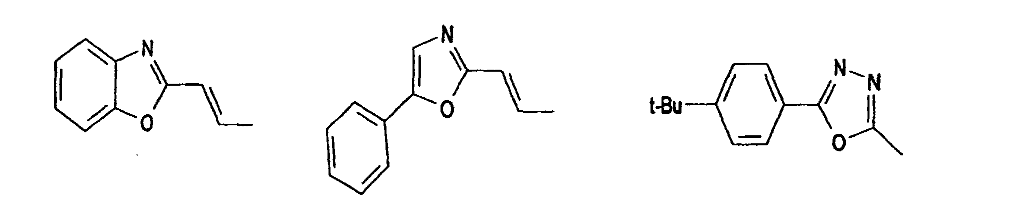 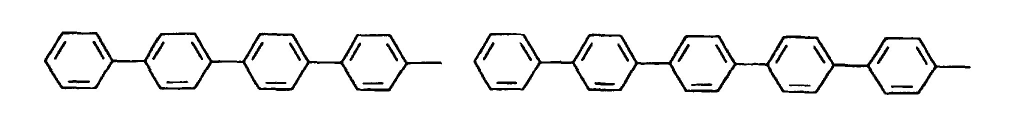 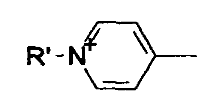 R' = C₁-C₂₂-Alkyl, C₂H₄SO₃-
IIIb) K = M = H und N = L und ist eine Gruppe der Formel: 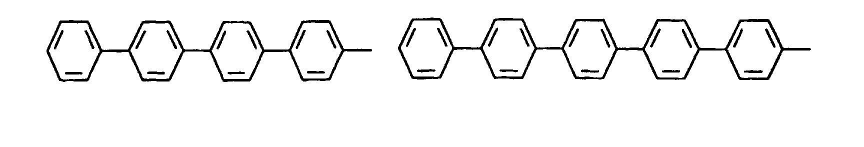 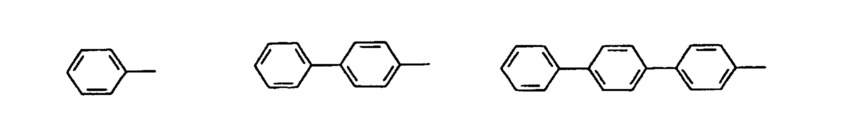 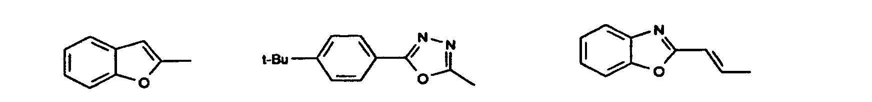 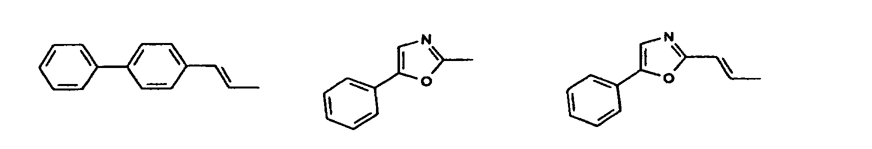 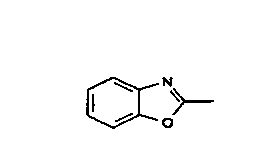
IIIc) K = M und ist eine Gruppe der Formel: 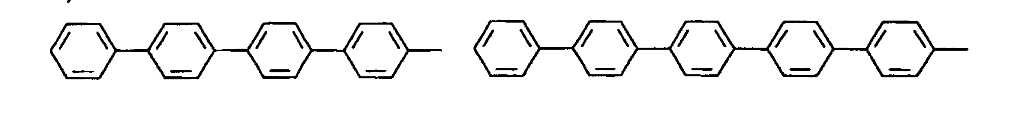 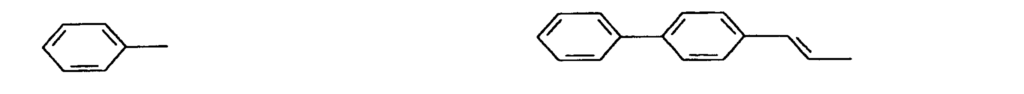 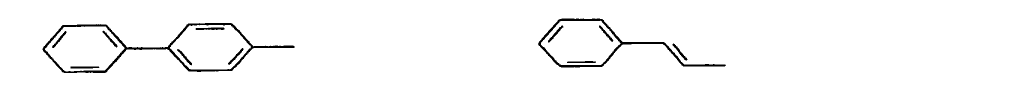 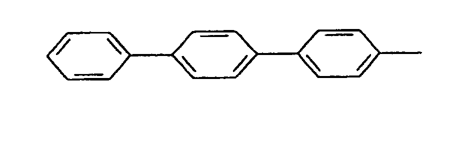 und N = L und ist eine Gruppe der Formel: 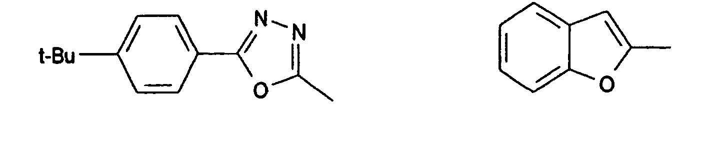 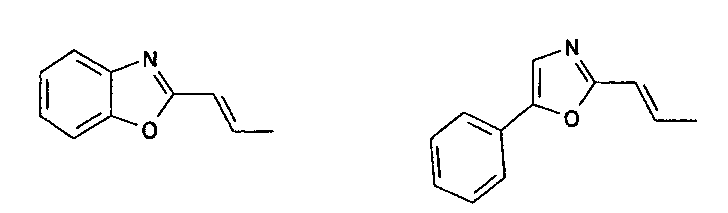 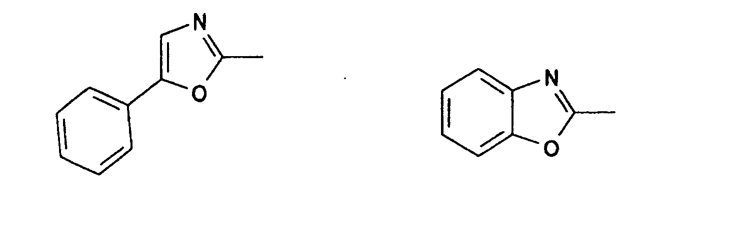 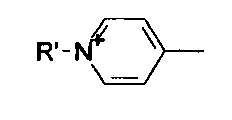 R'=C₁-C₂₂-Alkyl, C₂H₄SO₃⁻
IIId) K = M und ist eine Gruppe der Formel: 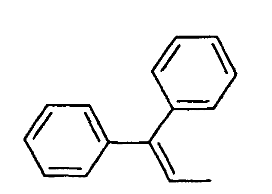 und N = L und ist die Gruppe der Formel: 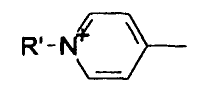 R'=C₁-C₂₂-Alkyl, C₂H₄SO₃⁻
IIIe) K = L = H und M = N und ist eine Gruppe der Formel: 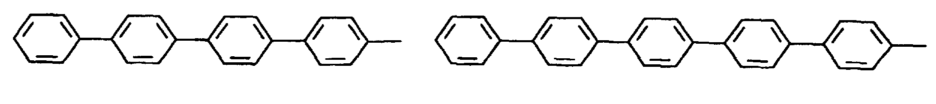 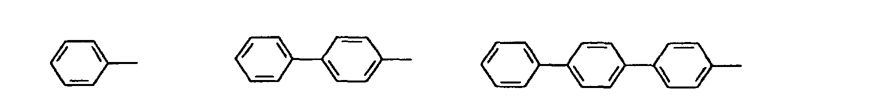 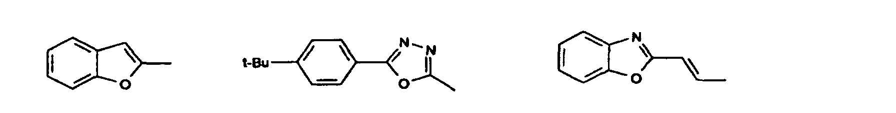 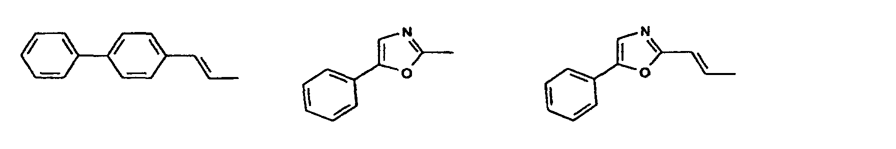 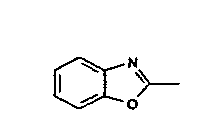
IIIf) K = L und ist eine Gruppe der Formel: 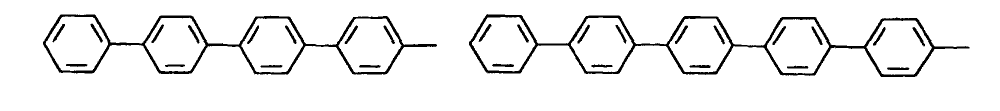 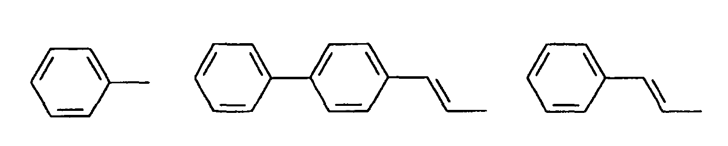 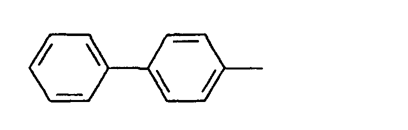 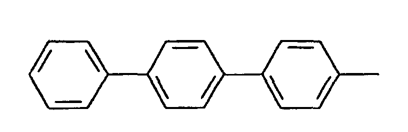 und M = N und ist die Gruppe der Formel: 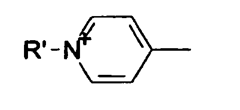 R'=C₁-C₂₂-Alkyl, C₂H₄SO₃⁻
eingesetzt wird.

3. Verwendung eines optischen Aufhellers gemäß Anspruch 1 oder 2 als Transformationsmaterial für UV-Licht zur Erhöhung des Wirkungsgrades in Solarzellen.

## Claims

1. Use of a spirobifluorene derivative having the Formula (III) 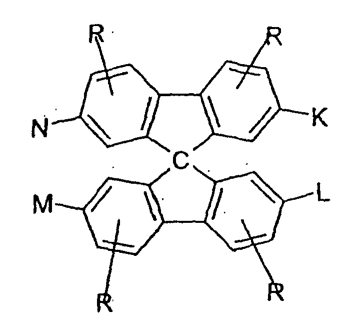 where K, L, M, and N are the same or different from one another and signify a group selected from the following formulae: 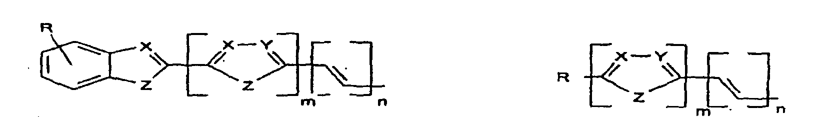 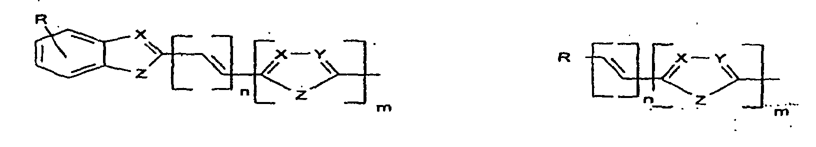 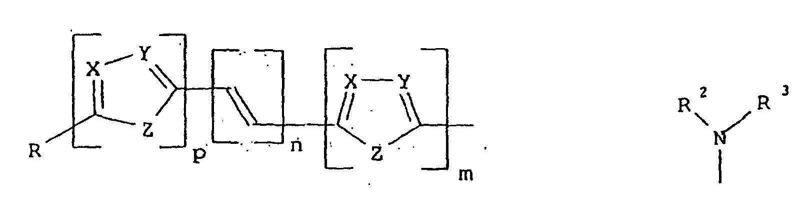 where
R is the same or different and stands for hydrogen, a linear or branched alkyl, alkoxy or carbo-alkoxy group with 1 to 22 C-atoms, -CN, - NO₂, -NR²R³, -N⁺R²R³R⁴, NOR²R³, -Ar or -O-Ar;
Ar is phenyl, biphenyl, 1-naphthyl, 2-naphthyl, 2-thienyl, 2-furanyl;
m, n, p are 0, 1,2 or 3,4,5;
X,Y are the same or different, selected from CR or N;
Z is -O-, -S-, -NR-, -CR¹R-, -CH=CH-, -CH=N-;
R¹ has the same significance as R;
R², R³, R⁴ are the same or different and stand for H, a linear or branched alkyl group with 1 to 22 C-atoms, -Ar, 3-methylphenyl
as an optical brightener.

2. Use in accordance with claim 1, **characterised in that** a spirobifluorene derivative is employed having the Formulae (IIIa to IIIg):
IIIa) K = L = M = N and is a group having the formulae 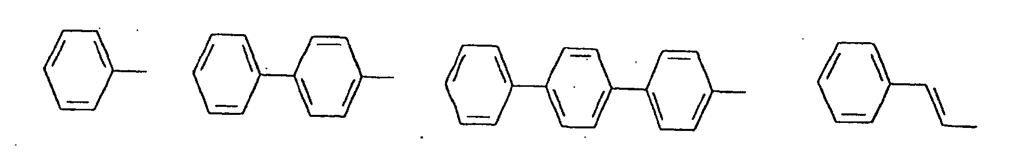 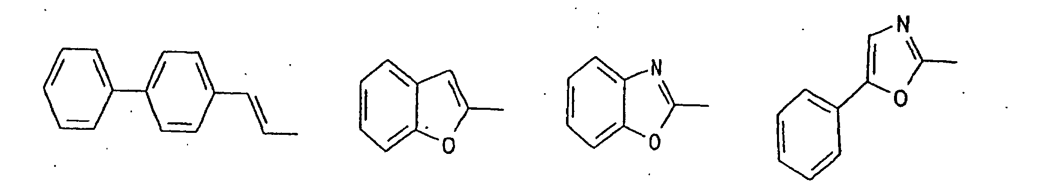 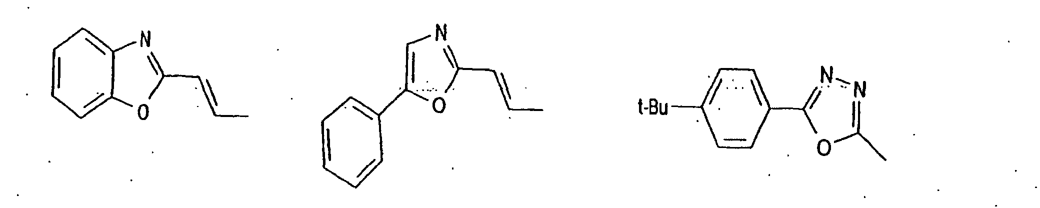 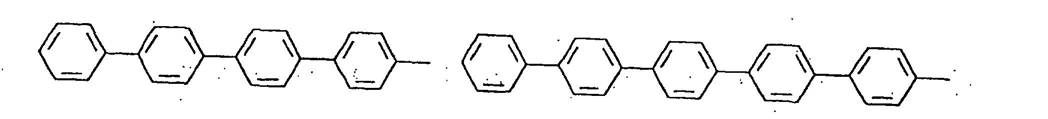 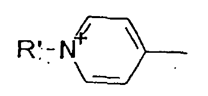 R' = C₁-C₂₂-Alkyl, C₂H₄SO₃₋
IIIb) K = M = H and N = L and is a group having the formulae 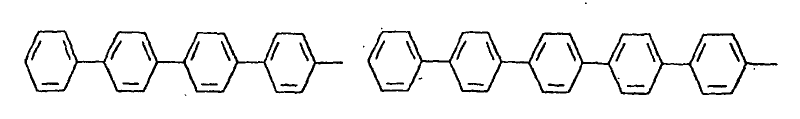 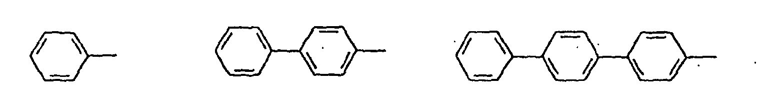 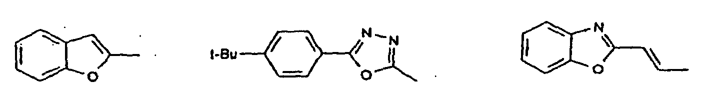 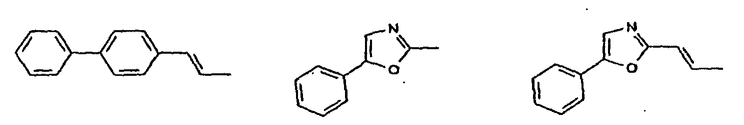 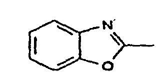
IIIc) K = M and is a group having the formulae: 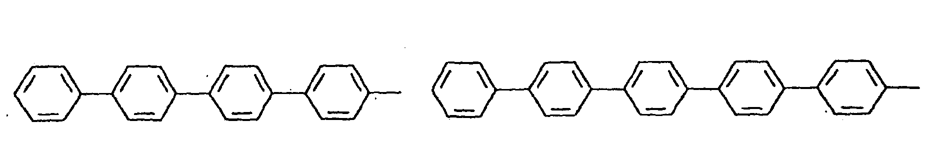 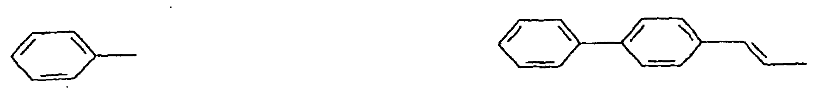 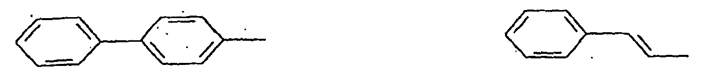 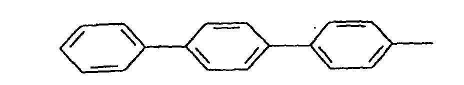 and N = L and is a group having the formulae: 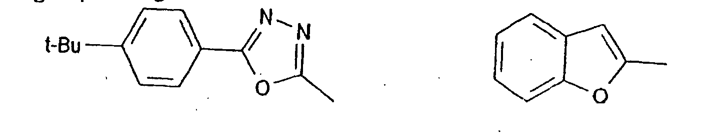 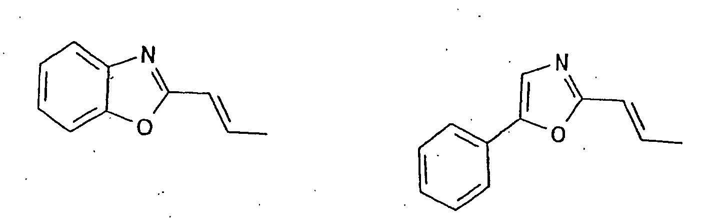 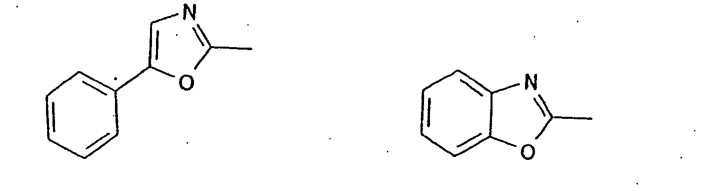 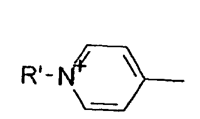 R¹ = C₁-C₂₂-alkyl, C₂H₄SO₃-⁻
IIId) K = M and is a group having the formula: 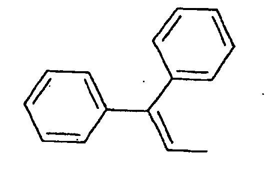 and N = L and is a group having the formula: 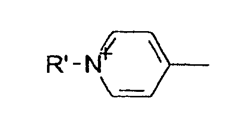 R¹ = C₁-C₂₂-alkyl, C₂H₄SO₃-⁻
IIIe) K = L = H and M = N and is a group having the formulae: 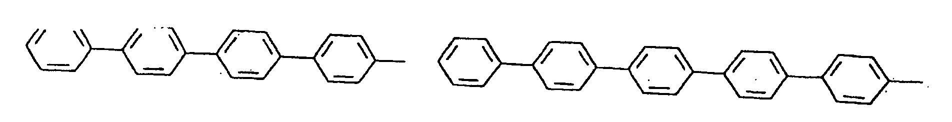 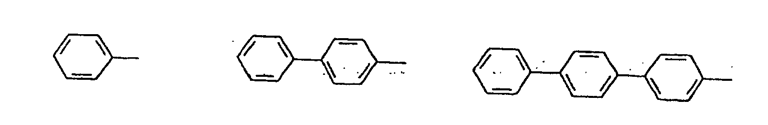 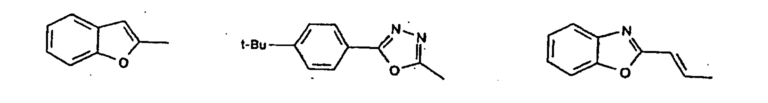 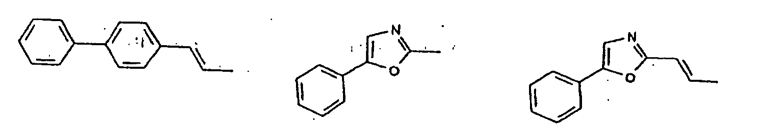 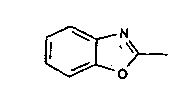
IIIf) K = L and is a group having the formulae: 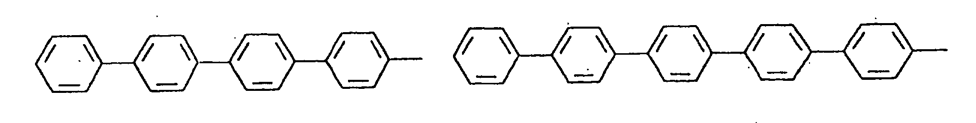 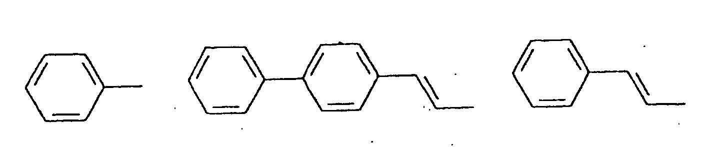 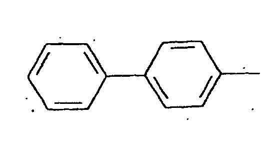 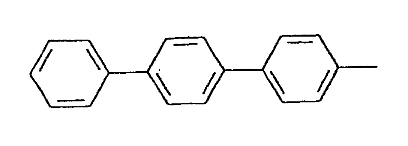 and M = N and is a group having the formula: 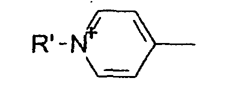 R¹ = C₁-C₂₂-alkyl, C₂H₄SO₃-⁻

3. Use of an optical brightener in accordance with claim 1 or 2 as a transformation material for UV light to increase the performance level in solar cells.

## Revendications

1. Utilisation d'un dérivé de spirobifluorène de formule (III) 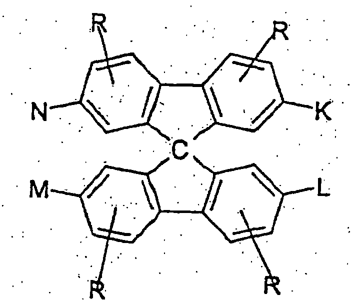 K, L, M, N sont identiques ou différents et représentent un groupe de formules suivantes : 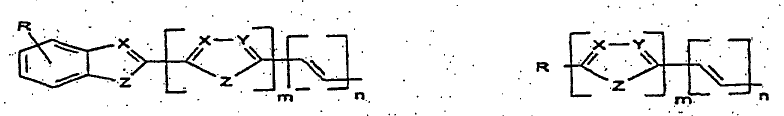 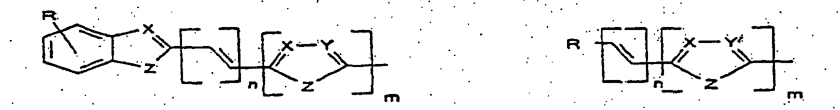 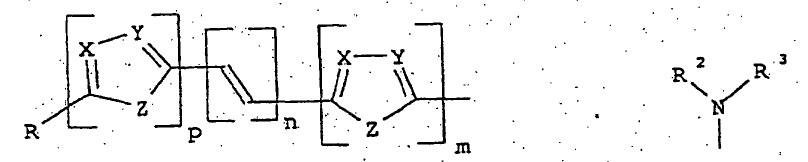
R identiques ou différents représentent un atome d'hydrogène, un groupe carbalkoxy, alkoxy ou alkyle linéaire ou ramifié présentant 1 à 22 atomes de carbone, -CN, -NO₂, -NR²R³, -N⁺R²R³R⁴, NOR²R³, -Ar ou -O-Ar ;
Ar représente un groupe phényle, biphényle, 1-naphtyle, 2-naphtyle, 2-thiényle, 2-furanyle,
m, n, p valent 0, 1, 2 ou 3, 4, 5 ;
X, Y identiques ou différents représentent CR ou N;
Z représente un groupe -O-, -S-, -NR-, -CR¹R-, -CH=CH-, -CH=N- ;
R¹ possède la même signification que R ;
R², R³, R⁴ sont identiques ou différents et représentent un atome d'hydrogène, un groupe alkyle linéaire ou ramifié présentant 1 à 22 atomes de carbone, -Ar, 3-méthylphényle,
en tant qu'azurant optique.

2. Utilisation selon la revendication 1, **caractérisée en ce qu'**on utilise un dérivé de spirobifluorène de formule (IIIa) à (IIIg) :
IIIa) K = L = M = N et représente un groupe de formule : 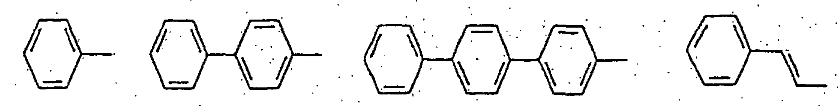 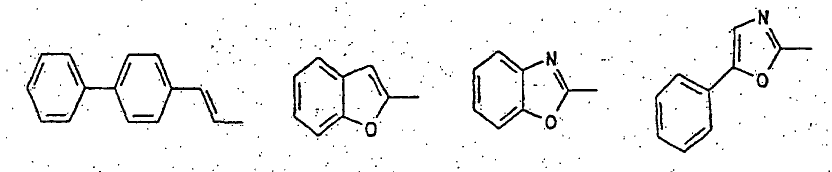 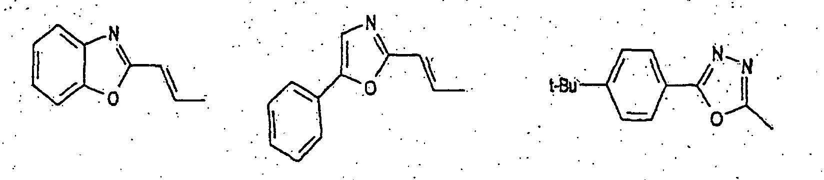 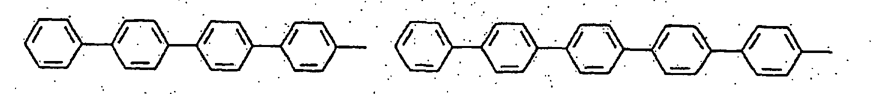 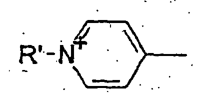 R' = alkyle en C₁-C₂₂, -C₂H₄SO₃⁻
IIIb) K = M = H et N = L et représente un groupe de formule : 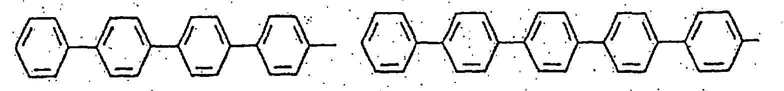 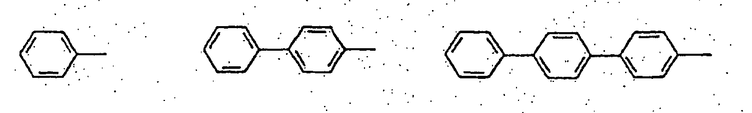 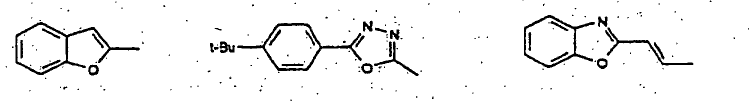 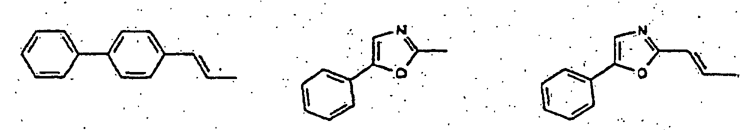 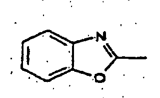
IIIc) K = M et représente un groupe de formule : 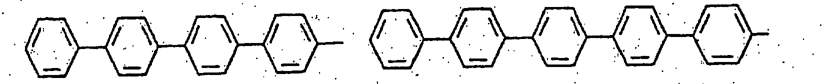 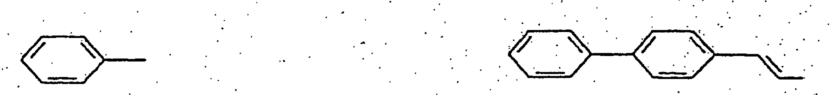 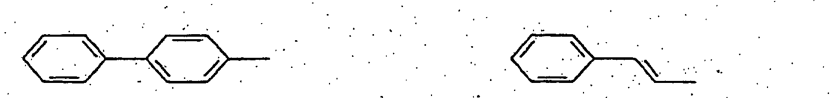 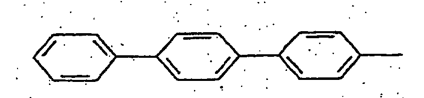 et N = L et représente un groupe de formule : 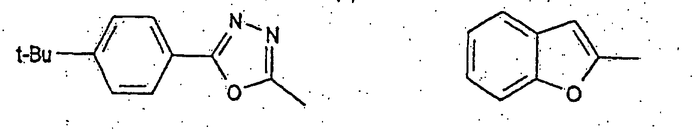 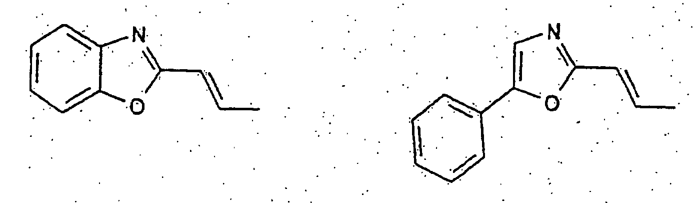 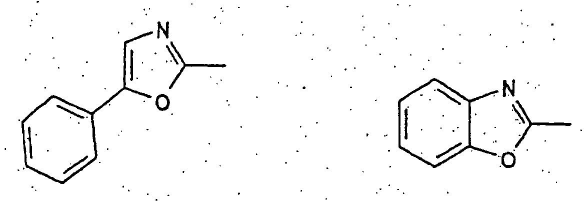 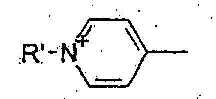 R' = alkyle en C₁-C₂₂, C₂H₄SO₃-
IIId) K = M et représente un groupe de formule : 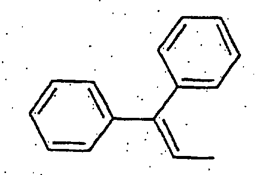 et N = L et représente un groupe de formule : 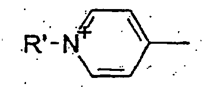 R' = alkyle en C₁-C₂₂, C₂H₄SO₃⁻
IIIe) K = M = H et N = L et représente un groupe de formule : 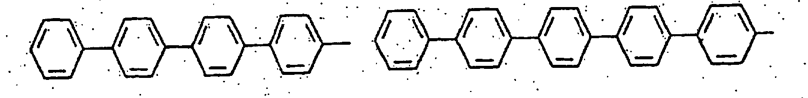 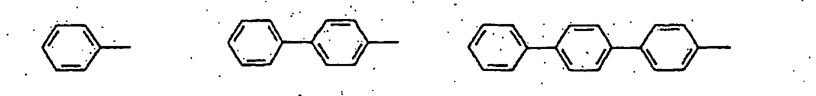 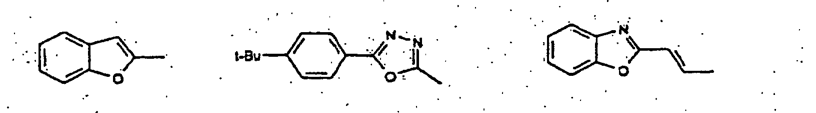 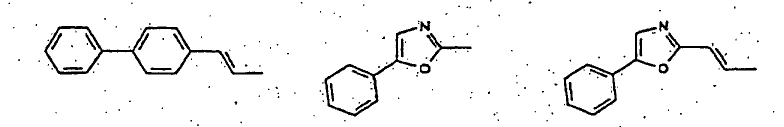 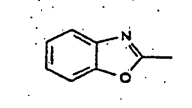
IIIf) K = L et représente un groupe de formule : 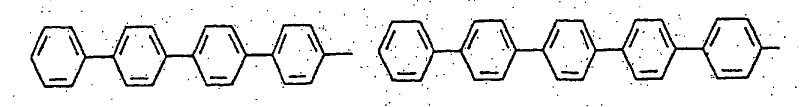 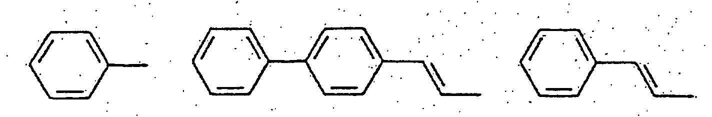 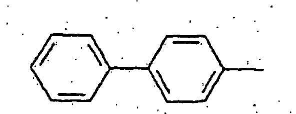 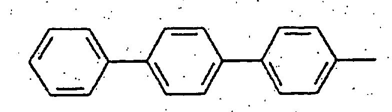 et M = N et représente un groupe de formule : 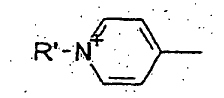
R' = alkyle en C₁-C₂₂, C₂H₄SO₃⁻

3. Utilisation d'un azurant optique selon la revendication 1 ou 2 en tant que matière de transformation de la lumière UV pour l'augmentation du degré d'efficacité dans les cellules solaires.
